(19) Europäisches Patentamt
European Patent Office
Office européen des brevets

(11) EP 3 669 189 B1

(12) # EUROPÄISCHE PATENTSCHRIFT

(45) Veröffentlichungstag und Bekanntmachung des Hinweises auf die Patenterteilung:
**13.12.2023 Patentblatt 2023/50**

(21) Anmeldenummer: **18749833.2**

(22) Anmeldetag: **10.08.2018**

(51) Internationale Patentklassifikation (IPC):
***G01N 33/497*** *(2006.01)* ***G01N 27/18*** *(2006.01)*
***G01N 33/00*** *(2006.01)*

(52) Gemeinsame Patentklassifikation (CPC):
**G01N 33/497; G01N 27/18; G01N 33/004**

(86) Internationale Anmeldenummer:
**PCT/EP2018/071838**

(87) Internationale Veröffentlichungsnummer:
**WO 2019/034570 (21.02.2019 Gazette 2019/08)**

(54) **SENSORANORDNUNG MIT DRUCKSENSOR UND THERMISCHEM GASSENSOR**

SENSOR ASSEMBLY COMPRISING A PRESSURE SENSOR AND A THERMAL GAS SENSOR

ENSEMBLE CAPTEUR COMPRENANT UN CAPTEUR DE PRESSION ET UN CAPTEUR DE GAZ THERMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.08.2017 EP 17186145**

(43) Veröffentlichungstag der Anmeldung:
**24.06.2020 Patentblatt 2020/26**

(73) Patentinhaber:
- **Hahn-Schickard-Gesellschaft für angewandte Forschung e.V.
78052 Villingen-Schwenningen (DE)**
- **GS Elektromedizinische Geräte G. Stemple GmbH
86916 Kaufering (DE)**

(72) Erfinder:
- **HEDRICH, Frank
78052 Villingen-Schwenningen (DE)**
- **KATTINGER, Gerhard
78122 St. Georgen (DE)**
- **STORZ, Matthias
78647 Trossingen (DE)**
- **BRONNER, Rolf
76532 Baden Baden (DE)**
- **BILLAT, Sophie
78052 Villingen-Schwenningen (DE)**

(74) Vertreter: **Burger, Markus et al
Schoppe, Zimmermann, Stöckeler
Zinkler, Schenk & Partner mbB
Patentanwälte
Radlkoferstraße 2
81373 München (DE)**

(56) Entgegenhaltungen:
**DE-A1-102010 047 159 US-A1- 2011 009 764
US-A1- 2017 176 405**

- **John Van Baar: "Distributed thermal micro sensors for fluid flow" In: "Distributed thermal micro sensors for fluid flow", 13. November 2002 (2002-11-13), Universiteit Twente, XP055524493, ISBN: 978-90-36-51828-4 Seite 11 - Seite 16**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Anmeldung bezieht sich auf eine Sensoranordnung, eine Sensorvorrichtung sowie auf ein Verfahren zur Herstellung einer Sensoranordnung, insbesondere auf Sensoranordnungen, Sensorvorrichtungen und Herstellungsverfahren für eine Sensoranordnung zur Bestimmung eines exspiratorischen $CO_2$-Gehalts von Atemluft.

Sensoranordnungen bzw. Sensorvorrichtungen können verwendet werden, um, zum Beispiel in der medizinischen Diagnostik, eine Atemluftanalyse durchzuführen. So ist beispielsweise der $CO_2$-Gehalt in der Ausatemluft eines Patienten eine wichtige Messgröße in der Anästhesie.

Herkömmliche $CO_2$ Sensoren, sogenannte Kapnometer, verwenden hauptsächlich infrarotspektroskopische Messverfahren, die eine Absorption an $CO_2$-Molekülen messen. Diese sogenannten nicht-dispersiven Infrarotsensoren (NDIR) messen die Konzentration von $CO_2$ im Atemgas durch eine Absorptions-Messung, genauer einer Infrarotabsorption bei 4,3 $\mu$m Wellenlänge. Derartige Sensoren sind nicht verbrauchend und ermöglichen eine Messung im Hauptstrom. Deren Funktionsweise bedingt einen entsprechend präzisen Aufbau, und umfasst teure optische Komponenten. Die verwendeten Komponenten sind sehr empfindliche gegenüber Verschmutzung durch Sekrete und Atemfeuchte, und die verwendete Fotodiode unterliegt einem Alterungsprozess.

Des Weitern kommen sogenannte Metalloxidsensoren (MOX) zum Einsatz, diese verwenden eine chemische Reaktion einer Dünnschicht und haben eine hohe Sensitivität bei kleinen Konzentrationen. Diese Sensoren sind kostengünstig, nachteilig ist dass der Sensor sich beim Betrieb verbraucht und keine Langzeitstabilität aufweist. Ebenso ist die Selektivität die Gasart betreffend gering, und derartige Sensoren sind nicht für $CO_2$ geeignet. Da die Betriebstemperatur bei bis zu 800 °C liegt ist der Einsatz im Hauptstrom riskant.

Sogenannte elektrochemisch potenziometrische Sensoren (NASICON) weisen eine hohe Genauigkeit bei geringen Dimensionen auf. Nachteilig ist, dass sich das Elektrodenmaterial verbraucht, und diese Sensoren verhältnismäßig teuer bei geringer Lebensdauer sind.

In der Druckschrift "Distributed thermal micro sensors for fluid flow" von John van Baar (ISBN 9-036-51828-8) werden thermische Sensor-Aktor-Strukturen für die Messung der Parameter Druck, dynamische Viskosität, Wärmeleitfähigkeit, spezifische Wärme, Dichte und die Strömungsgeschwindigkeit beschrieben. Dabei werden Beispiele für den Mehrwert von identischen einfachen Elementen und von mehreren Strukturen mit einer optimierten Geometrie für die Bestimmung eines Parameters offenbart. Die Platzierung der Strukturen in verschiedenen Umgebungen soll hier unterschiedliche Aufgaben erfüllen wobei eine kurze Beschreibung der thermischen Strömungssensoren, Drucksensoren und Wärmeleitfähigkeitssensoren vorliegt.

Des Weiteren gibt es mikroelektromechanische Drahtsensoren, sogenannte "MEMS" Drahtsensoren, die einen geringen Bauraum benötigen. Aufgrund deren physikalischen Messprinzips sind diese nicht verbrauchend und sind kostengünstig herzustellen. Ebenso vorteilhaft ist, dass die Messung im beruhigten Hauptstrom möglich ist. Nachteilig ist, dass solche Sensoren keine echte Gasanalytik anbieten, vielmehr müssen die Gaskomponenten bekannt sein. Auch ist die thermische Auflösung begrenzt und liegt bei 0,2 Vol.-% $CO_2$.

Um Messungen direkt am Patienten, sogenannte Point-of-Care Messungen, durchzuführen, existieren verschiedene tragbare Geräte für die Atemgasanalytik. Ein tragbarer Kapnograph zur $CO_2$-Messung aus Auswertung der mit einem Infrarotsensor arbeitet, wird beispielsweise von der Firma *Weinman Diagnostics* angeboten. Die Patientenanbindung erfolgt über eine Nasenbrille und die ausgeatmete Luft wird im Nebenstrom durch einen langen Schlauch zum Gerät zur Bestimmung des $CO_2$ Gehalts geführt.

Des Weiteren existieren verschiedene Systeme zur Patientenbeatmung, diese werden unterschieden je nach Einsatz im klinischen oder im Homecare-Bereich. Diese Systeme können Messeinrichtungen zur Bestimmung von Druck, Atemfluss und Atemgasanalyse umfassen, dazu müssen mehrere Geräte kombiniert werden, die überwiegend patientenfern messen. Daraus lässt sich ableiten, dass eine kostengünstige patientennahe Messung von Atemstrom und $CO_2$-Gehalt heute noch nicht umgesetzt ist.

Somit besteht die Aufgabe der vorliegenden Erfindung darin, ein Konzept für die Messung eines zu untersuchenden Gases mittels eines entsprechenden Sensors zu schaffen, das einerseits einfach und patientennah einsetzbar ist und andererseits eine hohe Messauflösung bei der Gasanalyse erfüllt.

Diese Aufgabe wird durch die Sensoranordnung nach Anspruch 1 gelöst.

Die Erfindung basiert auf der Erkenntnis, dass es vorteilhaft ist, die räumliche Entfernung zwischen Gassensor und Drucksensor zu minimieren, was den Vorteil hat, dass systematische Messfehler hierdurch so klein wie möglich ausfallen, da die Messwerte des Gassensors abhängig vom Druck sein können.

In einer ersten Ausführungsform umfasst der thermische Gassensor, beispielsweise als die für Gase durchlässige Messstruktur oder als Teil derselben, mindestens drei elektrische Leiterstrukturen, beispielsweise Drähte bzw. Siliziumdrähte, wobei die elektrischen Leiterstrukturen durch Zwischenräume beabstandet sind. Von diesen Leiterstrukturen ist eine erste elektrische Leiterstruktur, beispielsweise ein Draht, eingerichtet, um mit einem Heizsignal beaufschlagt zu werden, und eine zweite und eine dritte Leiterstruktur, beispielsweise Drähte, unsymmetrisch bezüglich der ersten Leiterstruktur angeordnet, z. B. derart, dass ein Abstand zwischen dem ersten Draht und dem zweiten Draht sich von dem Abstand zwischen dem ersten und dem dritten Draht unterscheidet, z. B. durch eine Anordnung auf unterschiedlichen Seiten des ersten Drahtes bzw. auf unterschiedlichen Seiten neben dem ersten Draht. Die zweiten und dritten Leiterstrukturen sind eingerichtet, um als Temperatursensoren zu arbeiten, so dass beispielsweise ein Unterschied in der Wärmeleitung durch das Gas bzw. Gasgemisch zwischen dem ersten und dem zweiten und der Wärmeleitung zwischen dem ersten und dritten Draht ermittelt werden kann, so dass basierend auf dem Unterschied der Wärmeübertragungen beispielsweise auf die Konzentration eines Anteils des Gasgemisches geschlossen werden kann.

Diese Ausführungsform basiert auf der Erkenntnis, dass es vorteilhaft ist, einen Gas-sensor mit drei asymmetrisch angeordneten Leiterstrukturen zu verwenden, da diese die thermische Wärmeübertragung sehr zuverlässig messen können.

In einer zweiten Ausführungsform sind die elektrischen Leiterstrukturen, z.B. Drähte bzw. Heizer/Detektoren, freitragend gespannt, so dass beispielsweise durch die Leerräume zwischen den Leiterstrukturen, z. B. Drähte, Gas zu der Einlassöffnung des barometrischen Drucksensors oder zu der druckempfindlichen Oberfläche des barometrischen Drucksensors gelangen kann.

Dieser Ausführungsform liegt die Erkenntnis zugrunde, dass es vorteilhaft ist, die Leiterstrukturen freitragend zu spannen, da dies durch das Nicht-Vorhandensein von Stützstrukturen den Drucksensor in seiner Arbeitsweise so gering wie möglich beeinflusst.

In einer dritten Ausführungsform sind die elektrischen Leiterstrukturen kristalline Siliziumdrähte, oder in einer weiteren Ausführungsform sind die elektrischen Leiterstrukturen ein polykristalliner Heizer auf einem Membranmaterial sowie Halbleiter-Temperatur-Detektoren oder Thermostapel. Dieser Ausführungsform liegt die Erkenntnis zugrunde, dass es vorteilhaft ist, bestimmte Materialien wie beispielsweise kristalline Siliziumdrähte als Material für die elektrischen Leiterstrukturen auszuwählen, die eine gute elektrische und thermische Leitfähigkeit aufweisen sowie einen hohen Temperatur-Koeffizienten des Widerstandes (TKR) und im Vergleich beispielsweise zu Platindrähten bei filigranem Durchmesser und kurzer Leitbahnlänge einen für elektrische Auswerteschaltungen vorteilhaften Grundwiderstand besitzen, die den Auswertestrom und damit die thermische Eigenerwärmung gering hält, um die Gasmessung möglichst schnell, d. h. ohne größere Verzögerung, ausführen zu können.

In einer vierten Ausführungsform umfasst der thermische Gassensor mindestens zwei elektrische Leiterstrukturen, wobei die elektrischen Leiterstrukturen durch zumindest einen Zwischenraum beabstandet sind. Eine erste elektrische Leiterstruktur ist hier eingerichtet, um mit einem Heizsignal beaufschlagt zu werden wobei eine zweite elektrische Leiterstruktur eingerichtet ist, um als Temperatursensor zu arbeiten.

In einer fünften Ausführungsform ist die Sensoranordnung ausgelegt um in einem ersten Zeitintervall die erste Leiterstruktur mit einem Heizsignal zu beaufschlagen und die zweite Leiterstruktur als Temperatursensor zu verwenden und weiterhin um in einem zweiten Zeitintervall die zweite Leiterstruktur mit einem Heizsignal zu beaufschlagen und die erste Leiterstruktur als Temperatursensor zu verwenden.

In einer sechsten Ausführungsform umfasst der thermische Gassensor beispielsweise als die für Gase durchlässige Messstruktur oder als Teil derselben mindestens drei elektrisch leitfähige Stege, wobei die Stege durch Zwischenräume beabstandet sind, und wobei eine Metallisierung oder Dotierung des ersten Stegs mit einem Heizsignal beaufschlagt wird und ein zweiter und ein dritter Steg unsymmetrisch bezüglich des ersten Stegs angeordnet sind, beispielsweise auf unterschiedlichen Seiten des ersten Stegs bzw. auf unterschiedlichen Seiten neben dem ersten Steg, und wobei Metallisierungen oder Dotierungen des zweiten und dritten Stegs eingerichtet sind, um als Temperatursensoren zu arbeiten.

Dieser Ausführungsform liegt die Erkenntnis zugrunde, dass es vorteilhaft ist, Stege als Leiterstrukturen zu verwenden, da diese mechanisch belastbar sind und somit einen guten Kompromiss zwischen Robustheit und minimaler Beeinträchtigung des Drucksensors bieten.

In einer siebten Ausführungsform sind die elektrischen Leiterstrukturen oder Drähte oder Stege von einem zu analysierenden Gas umgeben, wobei die erste elektrische Leiterstruktur oder der erste Draht oder der erste Steg eingerichtet ist, um einen Wärmeübertrag über das zu analysierende Gas, z. B. ein Gasgemisch, auf die zweite elektrische Leiterstruktur, den zweiten Draht oder den zweiten Steg und auf die dritte elektrische Leiterstruktur oder den dritten Draht oder Steg zu ermöglichen, und wobei die zweiten und dritten elektrischen Leiterstrukturen, Drähte oder Stege eingerichtet sind, um durch beispielsweise eine Auswerteeinrichtung als Sensoren für den Wärmeübertrag zu dienen.

Dieser Ausführungsform liegt die Erkenntnis zugrunde, dass es von Vorteil ist, die Gasmessung mittels Wärmeübertrag durchzuführen, da dies eine zuverlässige, verschleißfreie und schnelle Messung von Gasanteilen in einer Gasmischung ermöglicht.

In einer achten Ausführungsform wird das Heizsignal als ein periodisches Heizsignal bereitgestellt. Im Gegensatz zur statischen Heizeranregung lässt sich im periodischen Betrieb ein weiter Gasparameter extrahieren, z.B. neben der Wärmeleitfähigkeit die Temperaturleitfähigkeit des Gases. Die Temperaturleitfähigkeit a des Gases kann beispielsweise wie folgt bestimmt werden:

$$a = \frac{\lambda}{\rho * c_p}$$

wobei $\lambda$ der der Wärmeleitfähigkeit, $\rho$ der Dichte und $c_p$ der Wärmekapazität entspricht.

Dieser Ausführungsform liegt die Erkenntnis zugrunde, dass die Verwendung eines periodischen Heizsignals es ermöglicht, die Messung am zu analysierenden Gas sehr schnell mit einer guten Zeitauflösung durchzuführen, so dass es möglich ist, beispielsweise den $CO_2$-Gehalt eines Gasgemisches zeitaufgelöst und/oder mit hoher Genauigkeit zu messen.

In einer neunten Ausführungsform weist der thermische Gassensor ein Trägermaterial, beispielsweise ein Substrat wie Silizium auf. Das Trägermaterial kann ein Schichtmaterial sein, das beispielsweise auf dem barometrischen Drucksensor angeordnet ist. In dieser Ausführungsform weist der thermische Gassensor in einem zentralen Bereich eine durchgehende Ausnehmung, beispielsweise ein Loch, auf, die sich von einer dem Drucksensor abgewandten Oberfläche bis hin zu einer dem barometrischen Drucksensor zugewandten Oberfläche des Gassensors, beispielsweise des Trägermaterials, erstreckt, und wobei die für Gase durchlässige Messstruktur in einem Bereich der Ausnehmung, also beispielsweise in der Ausnehmung oder von dem Drucksensor aus gesehen in einer oberen Begrenzung der Ausnehmung, angeordnet ist, und z. B. den freitragend aufgespannten Heizer bzw. Heizdraht und die freitragend gespannten Detektoren bzw. Detektordrähte/Detektorelemente aufweist.

Dieser Ausführungsform liegt die Erkenntnis zugrunde, dass es von Vorteil ist, den thermischen Gassensor in unmittelbarer Nähe zu dem aktiven Bereich des Drucksensors anzuordnen, so dass der thermische Gassensor die Messung des Gases praktisch an dem gleichen Ort durchführt, an dem der Druck und die Gastemperatur durch den Drucksensor gemessen wird, wodurch systematische Fehler in den Messwerten bzw. der Messwertbetrachtung eliminiert werden können.

Erfindungsgemäß weist der thermische Gassensor einen Rahmen auf, der auf dem barometrischen Drucksensor angeordnet ist, und wobei der Rahmen die für Gase durchlässige Messstruktur trägt, so dass aktive Bereiche, wie z. B. Drähte bzw. freitragend gespannte Siliziumdrähte, freitragende Brückenstrukturen, wie beispielsweise ein freitragendes Heizelement und/oder freitragende Temperatursensorelemente, der Messstruktur einen freien Innenbereich, beispielsweise die durchgehende Ausnehmung bzw. ein Loch, des Gassensors, der von dem Rahmen umgeben ist, überspannen, und wobei die Gas-Einlassöffnung des Drucksensors oder die druckempfindliche Oberfläche, beispielsweise eine Membran, des Drucksensors an den freien Innenbereich des thermischen Gassensors angrenzt, so dass beispielsweise die Entfernung zwischen Druckmessmembran und den aktiven Bereichen der Messstruktur kleiner ist als das Dreifache der Länge eines der aktiven Bereiche oder kleiner ist als das Fünffache, oder kleiner ist als die größte Abmessung des freien Innenbereichs, beispielsweise Diagonale oder Durchmesser.

Dieser Ausführungsform liegt die Erkenntnis zugrunde, dass es vorteilhaft ist, einen Rahmen zu verwenden, um die Gasmessung des Gassensors räumlich möglichst nahe der Druckmessung durchzuführen, was durch den Rahmen ermöglicht wird, und wobei der Rahmen gleichzeitig den Gassensor mechanisch unterstützt. Der Rahmen kann gleichzeitig den Gas-Messraum (die Kaverne) nach außen abdichten, damit die benötigte Diffusionszeit bis zum vollständigen Ausgleich nach einer Gaskonzentrations-Änderung minimiert wird. Es ist darüber hinaus vorteilhaft, dass die Druckmessung, beispielsweise durch eine Druckmessmembran, in einer Entfernung von dem aktiven Bereich der Gasmessstruktur stattfindet, die kleiner ist als das Fünffache oder Dreifache der Länge der aktiven Bereiche oder kleiner ist als die größte Abmessung des freien Innenbereich, wodurch erreicht wird, dass die jeweiligen Messungen an praktisch dem gleichen Ort stattfinden, was zur Eliminierung von systematischen Messfehlern beiträgt.

In einer zehnten Ausführungsform ist der thermische Gassensor, beispielsweise das Trägermaterial oder der Rahmen, mittels eines Klebstoffs mit dem barometrischen Drucksensor verbunden, so dass der Klebstoff nicht mit der Gas-Einlassöffnung oder der druckempfindlichen Oberfläche des barometrischen Drucksensors Kontakt hat. Diesem Merkmal liegt der Erkenntnis zugrunde, dass es vorteilhaft ist, den thermischen Gassensor auf den Drucksensor zu kleben, da dies eine minimale mechanische Zusatzbelastung für den Drucksensor darstellt und somit der Drucksensor so gering wie möglich in seiner Funktion beeinträchtigt wird, insbesondere wenn sichergestellt ist, dass der Klebstoff nicht mit der Einlassöffnung oder der empfindlichen Oberfläche des Drucksensors in Kontakt ist.

In einer elften Ausführungsform weist die Sensoranordnung ein Leiterplattenmaterial, wie z. B. FR4, Flex oder Keramik, auf, und der Drucksensor und auf diesem der thermische Sensor sind auf einer Seite des Leiterplattenmaterials, oder beispielsweise einer Leiterplatte, angeordnet, und wobei auf der anderen, von dem Drucksensor und dem thermischen Sensor abgewandten Seite des Leiterplattenmaterials, oder beispielsweise der Leiterplatte, ein Stecker oder Lötkontakt zur elektrischen Kontaktierung angeordnet ist, oder wobei der Drucksensor und auf diesem der thermische Sensor in einer Ausnehmung des Leiterplattenmaterials, oder beispielsweise der Leiterplatte, angeordnet sind, und wobei auf einer anderen, von dem Drucksensor und dem thermischen Sensor abgewandten Seite des Leiterplattenmaterials ein Stecker oder Lötkontakt zur elektrischen Kontaktierung angeordnet ist, oder beispielsweise der Leiterplatte, angeordnet sind, und wobei auf einer Seite des Leiterplattenmaterials ein Stecker zur elektrischen Kontaktierung angeordnet ist, wobei beispielsweise eine Berandung der Ausnehmung des Leiterplattenmaterials einen Gasraum der Sensoranordnung begrenzt.

Diese Ausführungsform basiert auf der Erkenntnis, dass es vorteilhaft ist, die Sensoranordnung auf ein Leiterplattenmaterial aufzubringen, da dies eine vereinfachte Handhabung der Sensoranordnung ermöglicht, insbesondere wenn hierdurch zur Vereinfachung der Kontaktierung ein Stecker oder Lötkontakt angeordnet werden kann.

In einer zwölften Ausführungsform ist der barometrische Drucksensor ein mikroelektromechanischer, MEMS, Drucksensor, der beispielsweise ein barometrischer Höhenmesser sein kann.

Diese Ausführungsform basiert auf der Erkenntnis, dass es vorteilhaft ist, den Drucksensor als mikroelektromechanische Vorrichtung zu implementieren, da diese eine möglichst kompakte Bauweise ermöglicht. Des Weiteren ist von Vorteil, dass ein solcher MEMS Drucksensor ein geringes Gewicht aufweist, da es von Vorteil ist, bei einer patientennahen Messung eine Sensoranordnung zu verwenden, die möglichst wenig Gewicht aufweist, um den Patienten geringstmöglich zu belasten.

In einer dreizehnten Ausführungsform umfasst die Sensoranordnung eine Auswerteeinrichtung, die eingerichtet ist, um eine Gaskonzentration, beispielsweise eines Gasbestandteils des Gasgemisches, so z. B. eine $CO_2$-Konzentration basierend auf einer Phase und Amplitude von Sensorsignalen, beispielsweise Signalen von Sensordrähten oder Sensorstegen, die unter Verwendung des Gassensors erhalten werden, und abhängig von einer von dem Drucksensor gelieferten Druckinformation und ggf. Temperaturinformation zu ermitteln. Beispielsweise kann die Auswerteeinrichtung nicht auf der Leiterplatte selbst integriert sein, sondern von dieser getrennt sein, kann aber nach einer entsprechenden Miniaturisierung beispielsweise auf der Leiterplattenrückseite integriert werden.

Diese Ausführungsform basiert auf der Erkenntnis, dass es von Vorteil ist, die Gaskonzentration basierend auf Phase und Amplitude von Sensorsignalen zu ermitteln, da dies eine sehr schnelle Bestimmung der Gaskonzentration ermöglicht, beispielsweise schon nach einer einzigen Periodendauer des periodischen Sensorsignals, und ermöglicht, wenn mehrere Periodendauern des Sensorsignals in Betracht gezogen werden, eine wiederholte Messung der Gaskonzentration, wodurch man eine Reihe von Messwerten erhält, über die gemittelt werden kann, um einen statistisch relevanteren Messwert zu erhalten.

In einer vierzehnten Ausführungsform ist die Sensoranordnung von einem Gehäuse umgeben, das in seinem Inneren ein Volumen bereitstellt, in dem sich die Sensoranordnung befindet, wobei das Gehäuse eine Gehäuseöffnung, beispielsweise eine einzige Gehäuseöffnung, aufweist, durch die ein zu analysierendes Gas von der Außenseite des Gehäuses zu der sich in dem Volumen befindlichen Sensoranordnung durch einen Diffusionsvorgang gelangen kann, wobei beispielsweise die Gehäuseöffnung, die Messstruktur und die Gas-Einlassöffnung des barometrischen Drucksensors oder dessen druckempfindliche Oberfläche unmittelbar benachbart angeordnet sind.

Diese Ausführungsform basiert auf der Erkenntnis, dass es vorteilhaft ist, die Sensoranordnung von einem zur Umgebung gasdichten Gehäuse zu umgeben, in das das zu analysierende Gas durch einen Diffusionsvorgang gelangen kann, da der Diffusionsvorgang ein beruhigtes Gas darstellt, im Gegensatz zu einem Strömungsvorgang, bei dem das Gas zusätzlich zur Molekularbewegung eine Strömungsrichtung und Geschwindigkeit aufweist. Eine Messung in einem strömenden Medium ist fehlerbehafteter als die Messung an einem diffundierenden Medium, da durch ein strömendes Medium durch die Strömung Transportprozesse auftreten, insbesondere Wärmetransportprozesse aufgrund der Strömung, was die Messung der Gaskonzentration beeinträchtigen würde.

In einer fünfzehnten Ausführungsform weist die Öffnung des Gehäuses ein Gitter auf, das als mechanischer Schutz für die Sensoranordnung, und beispielsweise optional als Stützgitter für eine Membran, dient.

Dieser Ausführungsform liegt die Überlegung zugrunde, dass ein Gitter in oder vor der Gehäuseöffnung verhindern kann, dass makroskopische Partikel, beispielsweise Flüssigkeitströpfchen in der Ausatemluft, in die Sensoranordnung gelangen, was die Funktion der Sensoranordnung beeinträchtigen würde. Ein weiterer Vorteil ist, dass ein solches Gitter eine Membran stützen kann, eine solche Membran würde auch vor der Öffnung des Gehäuses angeordnet werden, um auch solche Partikel aufzuhalten, die durch ein Gitter alleine nicht von der Sensoranordnung ferngehalten werden können. Die Verwendung einer Membran ermöglicht es, beispielsweise Bakterien und/oder Viren von der Sensoranordnung fernzuhalten, so dass diese steril gehalten werden kann.

In einer sechzehnten Ausführungsform weist die Öffnung des Gehäuses eine Membran auf, die die Sensoranordnung vor einer Verschmutzung, beispielsweise durch Feuchtigkeit, Viren oder Bakterien, schützt und die Diffusion eines zu analysierenden Gases ermöglicht. Dieser Ausführungsform liegt die Erkenntnis zugrunde, dass die Verwendung einer Membran vorteilhaft ist, um die Sensoranordnung vor einer Verschmutzung zu schützen, d. h. diese steril zu halten, was in einem klinischen Umfeld unerlässlich ist, um die Sensoranordnung wiederholt zu verwenden, ohne dass diese gereinigt bzw. sterilisiert werden müsste.

In einer siebzehnten Ausführungsform weist das Gehäuse eine Öffnung auf, dies kann beispielsweise die einzige des Gehäuses sein, durch die, beispielsweise in einem betriebsfertigen Zustand der Sensoranordnung, das Gas in das Innere des Gehäuses gelangen kann. Beispielsweise wird hierdurch in dem Gehäuse ein strömungsberuhigtes Gebiet geschaffen, in dem sich der Gassensor befindet, so dass kein Durchfluss durch das Gehäuse stattfindet und so durch die für Gase durchlässige Messstruktur, beispielsweise durch die Ebene, in der die Drähte liegen, nur Gas durchtritt, das sich in die Druckkammer, die eine Sackdruckmesskammer sein kann, des barometrischen Drucksensors bewegt, beispielsweise hinein diffundiert, oder dass sich aus der Druckmesskammer des Drucksensors herausbewegt, beispielsweise durch Diffusion.

Diese Ausführungsform basiert auf der Erkenntnis, dass es vorteilhaft ist, ein strömungsberuhigtes Gebiet zu schaffen, in dem sich der thermische Gassensor befindet, da ein Durchfluss durch das Gehäuse, in dem sich der Sensor befindet, sich nachteilig auf die Messgenauigkeit der Sensoranordnung auswirken würde, da zum einen durch Wärmetransportprozesse der Gassensor in einer Funktion beeinträchtigt sein kann, und ebenso der Drucksensor einen falschen, in der Regel zu kleinen Druck messen würde.

Gemäß einer achtzehnten Ausführungsform der vorliegenden Erfindung, umfasst eine Sensorvorrichtung einen Strömungskanal, der beispielsweise hohlzylindrisch, z. B. ein Strömungsrohr/Tubus, sein kann, wobei der Strömungskanal in einer Wandung eine Öffnung aufweist, und eine Sensoranordnung gemäß einer der Ausführungsformen 1 bis 15 umfasst, wobei die Sensoranordnung durch die Öffnung mit dem Inneren des Strömungskanals räumlich verbunden ist, um einen Gasaustausch, beispielsweise durch Diffusion, zwischen dem Inneren des Strömungskanals und der Sensoranordnung zu ermöglichen.

Diese Ausführungsform basiert auf der Erkenntnis, dass es vorteilhaft ist, einen Strömungskanal mit einer Sensoranordnung zu kombinieren, wobei die Sensoranordnung durch eine Öffnung in der Wandung des Strömungskanals mit dem Inneren des Strömungskanals verbunden ist, um eine Gasmessung des sich in dem Strömungskanal befindenden Gases durchzuführen, da zum einen die Messung strömungsberuhigt nicht im Hauptkanal stattfindet, und die Sensoranordnung aufgrund deren räumlicher Anordnung als wiederverwendbares Modul, das leicht an dem Strömungskanal angebracht werden kann. In der Regel sind die Strömungskanäle preisgünstige Wegwerfartikel, die Sensoranordnung soll hingegen mehrfach verwendet werden. Durch die Anordnung der Sensoranordnung außerhalb des Strömungskanals wird eine einfache bauliche Trennung ermöglicht; des Weiteren wird gerade eben durch die seitliche Anordnung des Sensors erreicht, dass dieser nicht direkt in den Hauptkanal der Strömung eintaucht, sondern nur tangential mit der strömenden Ein- oder Ausatemluft in Kontakt kommt. Durch diese Bauart bedingt findet keine Strömung durch den Sensor bzw. die Sensoranordnung statt, sondern es kommt zu einem strömungsfreien Diffusionsvorgang des zu messenden Gases in die Sensoranordnung hinein, wodurch wie zuvor beschrieben, systematische Messungenauigkeiten wegfallen.

In einer neunzehnten Ausführungsform ist die Öffnung mit einer, beispielsweise bakterien- oder virenfilternden, Membran abgedeckt, wobei beispielsweise die Membran durch eine, beispielsweise poröse oder feinmaschige Gitterstruktur abgestützt wird, die in einer Öffnung eines die Sensoranordnung umgebenden Gehäuses angeordnet ist.

Diese Ausführungsform basiert auf der Erkenntnis, dass es von Vorteil ist, die Öffnung durch eine Membran abzudecken, um beispielsweise Bakterien oder Viren, die sich außerhalb der Sensoranordnung im Strömungskanal befinden können, nicht in die Sensoranordnung hineingelangen können, so dass der Sensor steril bzw. keimfrei bleibt, um wiederholt verwendet werden zu können, ohne dass es nötig ist, den Sensor zu reinigen bzw. zu sterilisieren. Da die Membran dünn genug sein soll, um eine Diffusion des zu analysierenden Gases zu ermöglichen, muss die Membran hinreichend dünn sein, wodurch diese mechanisch anfällig wird, daher ist es von Vorteil, die Membran durch eine Gitterstruktur abzustützen, um ein Zerreißen der Membran zu verhindern.

In einer zwanzigsten Ausführungsform diffundiert das zu analysierendes Gas, das sich im Innern der Sensorvorrichtung befindet, das beispielsweise durch den Strömungskanal fließt, durch die Membran hindurch zu der Sensoranordnung. Die Ausführungsform basiert auf der Erkenntnis, dass es vorteilhaft ist, das zu analysierende Gas in die Sensoranordnung hinein diffundieren zu lassen, um Messwertverfälschung aufgrund von Strömungseffekten zu vermeiden.

In einer einundzwanzigsten Ausführungsform ist der Abstand zwischen der Membran der Sensorvorrichtung und einer der Membran zugewandten Oberfläche des Gassensors kleiner als die Hälfte der größten Abmessung des Strömungskanals senkrecht zur mittleren Strömungsrichtung eines zu analysierenden Gases. Beispielsweise wird die mittlere Strömungsrichtung am Ort der Öffnung bzw. im Durchmesser bei einem runden Strömungskanal oder in der Diagonale bei einem rechteckigen Querschnitt des Strömungskanals betrachtet.

Diese Ausführungsform basiert auf der Erkenntnis, dass es vorteilhaft ist, den Gassensor möglichst nahe an der Membran zu positionieren, wobei der Abstand zwischen Membran und Sensor umso kleiner ist, je kleiner der Durchmesser des Strömungskanals ist, d. h. der Abstand skaliert mit dem Durchmesser des Strömungskanals. Ist der Abstand zwischen Membran und Gassensor möglichst gering, kann der Gassensor die Messung möglichst präzise und zeitnah durchführen, wodurch es zu einer möglichst genauen und unverfälschten Messung kommt.

In einer zweiundzwanzigsten Ausführungsform ist das die Sensoranordnung umgebende Volumen, das in Richtung des Strömungskanals durch die Öffnung, beispielsweise durch eine Membran, begrenzt ist, beispielsweise der von dem Strömungskanal aus gesehen hinter der Membran liegende Bereich der Sensoranordnung, kleiner als 1000, 500 oder 250 $mm^3$.

Die Ausführungsform basiert auf der Erkenntnis, dass es vorteilhaft ist, dass die Sensoranordnung umgebende Volumen zu begrenzen, auf ein möglichst kleinen Volumen, um eine weitestgehend vollständige Diffusion des zu messenden Gases in das Volumen in möglichst kurzer Zeit zu ermöglichen. Eine möglichst schnelle Diffusion ist wünschenswert, da das Messprinzip auf einer Phasenmessung eines periodischen Signales beruhen kann und die Periodendauer des Signales größer sein muss als die aparaturspezifische Diffusionszeit, um eine durch die Sensorgeometrie bedingte Beeinträchtigung des Zeitkurvenverlaufs des Messwertes zu minimieren. Der Rahmen kann gleichzeitig den Gas-Messraum (die Kaverne) nach außen abdichten, damit die benötigte Diffusionszeit bis zum vollständigen Ausgleich nach einer Gaskonzentrations-Änderung minimiert wird.

In einer dreiundzwanzigsten Ausführungsform ist die Sensorvorrichtung so ausgelegt, dass die Zeitdauer bis zum Ausgleich der Gaskonzentration im Bereich des Gassensors um höchstens 0,5 Vol.-% von der Gaskonzentration im Strömungskanal abweicht, kleiner als 10 ms ist. Dies kann beispielsweise erreicht werden durch eine geeignete Wahl von Membran und Gasvolumen in der die Sensoranordnung enthaltenden Kammer hinter der Membran.

Die Ausführungsform basiert auf der Erkenntnis, dass es von Vorteil ist, die Zeitdauer des Gaskonzentrationsausgleichs möglichst gering zu halten, um die Messung der Gaskonzentration in möglichst kurzer Zeit durchführen zu können. Es ist von Vorteil, dass die Zeit möglichst gering ist, da die Gaskonzentrationsmessung auf der Messung eines periodischen Zeitsignals beruhen kann, und wenn die Periodendauer des Zeitsignals in der Größenordnung der Zeitdauer liegt, die nötig ist, um die Gaskonzentration anzugleichen, kann eine derartige Messung nicht fehlerfrei durchgeführt werden.

In einer vierundzwanzigsten Ausführungsform stellt die die Sensoranordnung enthaltende Kammer ein strömungsberuhigtes Gebiet dar. Dies kann beispielsweise erreicht werden durch eine geeignete Anordnung der Öffnung und/oder mal der Geometrie der die Sensoranordnung enthaltende Kammer und/oder durch Wahl einer zwischen dem Strömungskanal und Kammer angeordneten Membran oder Filterstruktur. Die Kammer kann beispielsweise durch die Öffnung und optional durch eine Membran an den Strömungskanal angekoppelt sein. Das strömungsberuhigte Gebiet kann beispielsweise eine beruhigte Zone oder eine nahezu strömungsfreie Zone sein, so dass beispielsweise die für Gase durchlässige Messstruktur nicht in einem Strömungsbereich liegt.

Diese Ausführungsform basiert auf der Erkenntnis, dass es von Vorteil ist, die Sensoranordnung in einem strömungsberuhigten Gebiet anzuordnen, da Strömungseffekte einen negativen Einfluss auf die Messgenauigkeit der Sensoranordnung haben können, da zum einen Strömungen die Arbeitsweise des Temperaturgassensors beeinträchtigen, und ebenso die Druckmessung verfälschen können.

In einer fünfundzwanzigsten Ausführungsform weist die Sensorvorrichtung einen Strömungssensor auf, der eine Strömungsgeschwindigkeit und/oder einen Gasmassestrom und/oder einen Volumenstrom in dem Strömungskanal ermitteln kann, beispielsweise kann dieser als Sensorfinger, der in den Strömungskanal ragt, ausgeführt sein.

Diese Ausführungsform basiert auf der Erkenntnis, dass es vorteilhaft ist, einen Strömungssensor in der Sensorvorrichtung vorzusehen, da durch diesen Messgrößen erfasst werden können, die eine Aussage darüber erlauben, ob der Gassensor unter den gemessenen Bedingungen, wie beispielsweise Strömungsgeschwindigkeit, aussagekräftig ist. So könnte beispielsweise in dem Fall einer sehr großen Strömungsgeschwindigkeit die Strömungsfreiheit innerhalb der Sensoranordnung nicht garantiert sein, wodurch die Messwerte verfälscht sein können. In diesem Fall könnte man potentiell verfälschte Messwerte verwerfen, wenn der Strömungssensor eine Strömungsgeschwindigkeit feststellt, die oberhalb einer gewissen Grenze liegt.

In einer sechsundzwanzigsten Ausführungsform weist die Sensorvorrichtung einen zweiten barometrischen Drucksensor auf, der einen Umgebungsdruck misst. Die Sensorvorrichtung ist beispielsweise eingerichtet, um den Atemdruck abhängig von einer Differenz zwischen den Druckwerten des ersten und zweiten Drucksensors zu bestimmen.

Diese Ausführungsform basiert auf der Erkenntnis, dass es von Vorteil ist, den Atemdruck zu ermitteln, unter Verwendung des Druckwerts des barometrischen Drucksensors und des zweiten barometrischen Drucksensors, um beurteilen zu können, ob die Gasanteilsmessung, die von der Sensoranordnung ausgeführt wird, ein gültiges Messergebnis liefert. Im Fall, dass der Atemdruck außerhalb eines bestimmten Bereiches liegt, kann die Gasmessung unter Umständen nicht akkurat sein.

In einer siebenundzwanzigsten Ausführungsform erkennt die Sensorvorrichtung einen Zeitpunkt für eine Kalibrierung basierend auf einer Information über die Strömungsgeschwindigkeit im Strömungskanal und/oder auf einer Information über eine Strömungsrichtung im Strömungskanal, z. B. wenn erkannt wird, dass genügend Frischluft oder mit Anästhesiegas angereicherte Frischluft angesaugt oder appliziert wurde, um entsprechend darauf eine Kalibriedung, beispielsweise eine Neukalibrierung, des thermischen Gassensors durchzuführen. Diese Ausführungsform basiert auf der Erkenntnis, dass es von Vorteil ist, die Sensorvorrichtung wenn nötig zu kalibrieren bzw. neu zu kalibrieren, und dass Informationen über die Strömungsgeschwindigkeit oder der Strömungsrichtung im Strömungskanal gute Kriterien sind, um zu entscheiden, ob eine solche Kalibrierung durchgeführt werden soll.

In einer achtundzwanzigsten Ausführungsform gibt die Sensorvorrichtung eine Warnung zum Zeitpunkt der Kalibrierung aus, entsprechend auf einer Erkennung einer zu hohen Konzentration, beispielsweise höher als ein Schwellwert, eines bestimmten Gasanteils.

Diese Ausführungsform basiert auf der Erkenntnis, dass es von Vorteil ist, wenn ein zu hoher Gasanteil zum Zeitpunkt der Kalibrierung festgestellt wird eine Warnung auszugeben, damit erkannt werden kann, dass die Kalibrierung unter Umständen nicht akkurat durchgeführt wurde, und ggf. die Kalibrierung erneut durchgeführt werden kann, unter Umständen unter bestimmten Randbedingungen, beispielsweise nach einem Durchspülen der Sensorvorrichtung mit einem bestimmten Gasgemisch.

Gemäß einer neunundzwanzigsten Ausführungsform ist ein Verfahren zur Herstellung einer Sensoranordnung gegeben, wobei das Verfahren das Bereitstellen eines barometrischen Drucksensors und eines thermischen Gassensors, sowie das Fixieren des thermischen Gas-sensors, beispielsweise durch Kleben, auf dem barometrischen Drucksensor umfasst, so dass eine für Gase durchlässige Messstruktur des thermischen Gassensors, beispielsweise unmittelbar, vor einer Gas-Einlassöffnung des barometrischen Drucksensors oder vor einer druckempfindlichen Oberfläche, beispielsweise einer Membran, des barometrischen Drucksensors angeordnet ist.

Gemäß einer dreißigsten Ausführungsform ist ein Verfahren zum Betrieb einer Sensorvorrichtung gegeben, wobei das Verfahren ein Erkennen eines Zeitpunkts für eine Kalibrierung basierend auf einer Information über ein Strömungssignal und/oder über eine Strömungsrichtung in dem Strömungskanal aufweist, wenn beispielsweise erkannt wird, dass genügend Frischluft oder mit Anästhesiegas angereicherte Frischluft angesaugt wurde, und ein Durchführen einer Kalibrierung, beispielsweise einer Neukalibrierung, eines thermischen Gassensors entsprechend darauf, beispielsweise auf das Erkennen eines Zeitpunkts für die Kalibrierung, umfasst.

Fig. 1 zeigt eine schematische Darstellung einer Sensoranordnung gemäß einer Ausführungsform.

Wie zuvor beschrieben ist es für bestimmte Anwendungen notwendig einen $CO_2$-Gehalt von Gasen bzw. Gasgemischen zu bestimmen. Fig. 1 zeigt eine mögliche Ausführungsform einer Sensoranordnung, die geeignet ist, um Eigenschaften von Gasen zu messen. Die Sensoranordnung umfasst einen barometrischen Drucksensor 10 und einen thermischen Gassensor 20. Der thermische Gassensor ist auf dem barometrischen Drucksensor 10 angeordnet und weist eine für Gase durchlässige Messstruktur 22, die beispielsweise ein Loch sein kann, auf, wobei die Messstruktur derart angeordnet ist, dass sich diese vor der druckempfindlichen Oberfläche oder der Gaseinlassöffnung des Drucksensors befindet. Dadurch, dass sich der Drucksensor in unmittelbarer Nähe zu dem Gassensor befindet, kann sichergestellt werden, dass sowohl die Druckmessung als auch die Messung des Gases im Wesentlichen am gleichen Ort stattfinden. Dies ist von Vorteil, da die Messwerte des thermischen Gassensors 20 unterschiedlich ausfallen können, je nachdem welcher Druck und welche Temperatur im Bereich des thermischen Gassensors vorliegt. Ist beispielsweise die Druck- und Temperaturabhängigkeit der Messwerte des thermischen Gassensors 20 bekannt, können diese gegebenenfalls angepasst bzw. entsprechend interpretiert werden. Dies trägt zur Steigerung der Aussagekraft der Messwerte des thermischen Gassensors 20 bei.

Solch eine Anordnung 100 stellt ein miniaturisiertes Sensorsystem dar, das zur Konzentrationsbestimmung von Gasen bzw. Gasgemischen, beispielsweise bei der patientennahen Bestimmung einer $CO_2$-Konzentration in Expirationsgas bei der Ausatmung dar, welches als ein sogenanntes Chip Scale Package ausgeführt sein kann. Ein solches Chip Scale Package ist im Allgemeinen ein Gehäuse in der Größenordnung eines elektronischen Chips und umfasst in diesem Fall einen Drucksensor sowie einen Gassensor. Diese Sensoranordnung 100 kann des Weiteren eine gasdichte Messkammer umfassen, und kann an einen Strömungskanal, durch den beispielsweise das Atemgas eines Patienten geleitet wird, angebunden sein, beispielsweise über eine Seitenbohrung eines solchen Kanals.

Obwohl die Sensoranordnung 100 in Fig. 1 in einer quadratischen Ausführung gezeigt ist, versteht es sich, dass diese rechteckig, rund oder auch vieleckig ausgeführt sein kann. Ähnliches gilt für die durchlässige Messstruktur 22, die in Fig. 1 quadratisch gezeigt ist. Es ist ebenso selbstverständlich, dass diese Messstruktur auch rechteckig, vieleckig, rund oder auch oval sein kann oder auch unregelmäßig geformt sein kann.

Fig. 2 zeigt eine weitere Ausführungsform der Sensoranordnung, wie diese schon im Zusammenhang mit Fig. 1 beschrieben wurde. Die Sensoranordnung 200 weist einen barometrischen Drucksensor 10 und einen thermischen Gassensor 20 auf. In dieser Ausführungsform umfasst der thermische Gassensor drei elektrische Leiterstrukturen 30, 32 und 34. Die Leiterstrukturen überspannen die Bereich der durchlässigen Messstruktur 22. Der die für Gase durchlässige Messstruktur 22 überspannender Teil der Leiterstrukturen 30, 32, 34 kann als Draht oder Steg ausgeführt sein. In einer Ausführungsform ist der mittlere Draht 30 eingerichtet, um mit einem Heizsignal beaufschlagt zu werden, die beiderseitig von dem mittleren Draht angeordneten Drähte 32 und 34 sind in unterschiedlichen Abständen zu dem mittleren Draht 30 angeordnet und dienen als Temperatursensoren. Die erwähnten Drähte können beispielsweise kristalline Siliziumdrähte sein.

Der in Fig. 2 gezeigte thermische Gassensor umfasst in diesem Beispiel drei freitragendgespannte filigrane kristalline Siliziumdrähte, die von dem zu analysierenden Gas umgeben sind. Die Drähte können zwischen einem Rahmen, der diese trägt, gespannt sein. Der mittlere Draht kann mit einem Heizsignal beaufschlagt werden, dies kann ein periodisches Heizsignal sein. Die beiden relativ zum Heizdraht links und rechts unsymmetrisch angeordneten Drähte arbeiten als Temperatursensoren. Diese Temperatursensoren messen den Wärmeübertrag von dem Heizdraht auf die Sensordrähte, der Wärmeübertrag erfolgt über die an sich unbekannten Wärmeübergänge vom Heizdraht in das zu analysierende Gas und von diesem Gas auf die Sensordrähte. Durch die Messung der Temperaturantwort mit zwei Sensordrähten, die im Allgemeinen identisch sind, aber in unterschiedlichen Abständen zu dem Heizdraht angeordnet sind, lassen sich die unbekannten Wärmeübergänge in der Messanordnung eliminieren. Durch Messung von Phase und Amplitude der beiden Sensorsignale in den beiden Sensordrähten kann auf die Bestandteile des Gases bzw. des Gasgemisches zurückgeschlossen werden, die Phase und Amplitude sind im Wesentlichen von dem Wärmeübertrag durch das Gas abhängig.

Wie zuvor erwähnt, kann das Sensorsystem als ein Chip Scale Package ausgeführt sein und umfasst in diesem Fall, wie in Fig. 3 gezeigt, eine Leiterplatte 40, einen barometrischen Drucksensor 10, der ein mikroelektomechanischer (MEMS) Drucksensor mit beispielsweise 24 Bit Auflösung sein kann, wie beispielsweise ein barometrischer Höhenmesser und umfasst einen darüber geklebten thermischen Sensor 20, der ebenfalls ein mikroelektomechanisches Bauteil sein kann.

Zur Kontaktierung des Sensorsystems 200 umfasst die runde Leiterplatte Kontaktierungsvorrichtungen 42, die mittels Bondingdrähten eine Kontaktierung der elektrischen Leiterstrukturen der Sensoranordnung 200 ermöglichen. Des Weiteren kann die Leiterplatte elektrische Bauteile wie Kondensatoren, Dioden oder aktive elektronische Bauelemente 44 umfassen, die beispielsweise beim Betrieb des Sensorsystems Verwendung finden. Es können ganze miniaturisierte Auswerteschaltungen auf dem Modul angeordnet sein.

Wie in Fig. 4 gezeigt, kann auf die Rückseite der Leiterplatte 40 ein Stecker zur elektrischen Kontaktierung angeordnet sein. Dieser Stecker, der beispielsweise ein Fine-Pitch-Stecker, ein Federkontakt-System oder eine feste Lötverbindung sein kann, ermöglicht eine standardisierte und leichte Kontaktierung des Sensorsystems. Das gesamte Sensorsystem kann beispielsweise von einem Gehäuse umschlossen sein, wie in Fig. 6 gezeigt ist. Fig. 6 zeigt die Sensoranordnung mit Leiterplatte und rückseitigem Stecker, welche von einem Gehäuse umschlossen ist.

Fig. 5 zeigt den gesamten Sensorstapel, wie dieser in Fig. 4 gezeigt ist, einmal in seitlicher Ansicht, in einer Draufsicht und in einer steckerseitigen Ansicht. Die Dimensionsangaben sind in Millimetern, wodurch ersichtlich wird, dass das Sensorsystem eine sehr kleine Bauform aufweist. So beträgt der Durchmesser der Leiterplatte 8 mm und die Kantenmaße des Drucksensors liegen bei etwa 2,6 mm. So ergibt sich eine sehr kleine Bauform von beispielsweise ca. 1 $cm^3$ bezogen auf die Außenabmessungen. Ist der Sensor noch mit einem Gehäuse umgeben, ist der Gasmessraum beispielsweise ca. 250 $mm^3$ groß. Hierbei bietet die Sensoranordnung die Messung von Druck, Temperatur und Gaskonzentration, beispielsweise die $CO_2$-Konzentration. Die Messung von Druck und Temperatur erfolgt beispielsweise im Gassensor bzw. im sogenannten $CO_2$-Modul. Hierdurch ist eine genaue Driftkorrektur, beispielsweise einer $CO_2$-Konzentrationsmessung, möglich.

Fig. 7 zeigt eine Ausführungsform der Sensoranordnung mit einem Strömungskanal 700, der als einmalig verwendbarer Beatmungstubus (disposable flowtubus) ausgebildet ist. Die gezeigte Vorrichtung dient beispielsweise zur Konzentrationsbestimmung von Gasen, vorliegend zur patientennahen Bestimmung der $CO_2$-Konzentration in der vom Patienten ausgeatmeten Luft. Der Strömungskanal 700 weist in einer Wandung eine Öffnung auf wobei die als $CO_2$-Modul ausgebildete Sensoranordnung 100 in einer Messkammer angeordnet ist. Die Sensoranordnung ist durch die Öffnung mit dem Inneren des Strömungskanals 700 räumlich verbunden, um einen Gasaustausch zwischen dem Inneren des Strömungskanals 700 und der Sensoranordnung zu ermöglichen. An seiner Öffnung zum Strömungskanal 700 ist eine Gitterstruktur 702 angeordnet, durch den der Gasaustausch erfolgt. Während einer Einatmungsphase des Patienten (Inspiration 704) erfolgt eine Umströmung des $CO_2$-Moduls in der Messkammer mit frischer Luft oder einem Kalibriergas. Während einer Ausatmungsphase des Patienten erfolgt eine Umströmung der Sensoranordnung mit der ausgeatmeten Luft, wobei die $CO_2$-Konzentration in der vom Patienten ausgeatmeten Luft gemessen werden kann.

Durch die seitliche Anordnung des Sensors taucht dieser nicht direkt in den Hauptkanal der Strömung ein, sondern nur tangential mit der strömenden Ein- oder Ausatemluft in Kontakt kommt. Durch diese Bauart bedingt findet keine Strömung durch den Sensor bzw. die Sensoranordnung statt, sondern es kommt zu einem strömungsfreien Diffusionsvorgang des zu messenden Gases in die Sensoranordnung hinein, wodurch systematische Messungenauigkeiten wegfallen.

Die Öffnung zur Messkammer ist durch eine Membran abgedeckt, um Bakterien oder Viren, die sich außerhalb der Sensoranordnung im Strömungskanal 700 befinden können, nicht in die Sensoranordnung hineingelangen können, so dass der Sensor steril bzw. keimfrei bleibt, um wiederholt verwendet werden zu können.

Die Sensorvorrichtung aus Fig. 7 weist weiterhin einen Strömungssensor 706 auf, der eine Strömungsgeschwindigkeit und/oder einen Gasmassestrom 708 und/oder einen Volumenstrom in dem Strömungskanal 700 ermitteln kann. Durch diesen als Sensorfinger ausgebildete Strömungssensor 706, der in den Strömungskanal 700 ragt, können Messgrößen erfasst werden, die eine Aussage darüber erlauben, ob der Gassensor unter den gemessenen Bedingungen, wie beispielsweise Strömungsgeschwindigkeit, aussagekräftig ist. So könnte beispielsweise in dem Fall einer sehr großen Strömungsgeschwindigkeit die Strömungsfreiheit innerhalb der Sensoranordnung nicht garantiert sein, wodurch die Messwerte verfälscht sein können.

Nachfolgend sind weitere Ausführungsbeispiele und Aspekte der Erfindung dargestellt: Es sei darauf hingewiesen, dass die Ausführungsbeispiele für sich genommen eingesetzt werden können. Ferner können die verschiedenen Merkmale, Funktionalitäten und Details der einzelnen Ausführungsbeispiele auch in anderen Ausführungsbeispielen eingesetzt werden, soweit nicht zwingende technische Gründe dem entgegenstehen.

Eine Ausführungsbeispiel bezieht sich auf ein miniaturisiertes Sensorsystem zur Konzentrationsbestimmung von Gasen, beispielsweise zur patientennahen Bestimmung der $CO_2$-Konzentration im Exspirationsgas bei der Ausatmung, welches als Chip-Scale Package (Chip-Größen-Gehäuse) ausgeführt ist, das mit einer gasdichten Messkammer und über eine Seitenbohrung an den Haupt-Strömungs-Kanal angebunden ist.

Das Chip-Scale Package (Chip-Größen-Gehäuse) aus Fig. 4 umfasst beispielsweise eine runde Leiterplatte, einen barometrischen MEMS (mikroelektromechanischen) Drucksensor (beispielsweise mit 24bit Auflösung, barometrischer Höhenmesser) und einen beispielsweise darüber geklebten thermischen Sensor (MEMS bzw. mikroelektromechanisch, Hahn-Schickard). Rückseitig ist beispielsweise ein Fine-Pitch-Stecker, ein Federkontakt-System oder eine feste Lötverbindung zur elektrischen Kontaktierung angeordnet. Der Sensorstapel wird beispielsweise mit einem RP (Rapid-Prototyping; Schnelle Prototypenerstellung) Gehäuse umschlossen, das an seiner Öffnung zum Strömungsrohr beispielswese eine Gitterstruktur und eine Vertiefung zur Aufnahme eines gegen Leckage abdichtenden O-Rings aufweist.

Zu den Vorteilen einer solchen Anordnung zählen beispielsweise: eine sehr kleine Bauform: beispielsweise ca. 1 $cm^3$. (Außenabmessungen) mit einem Gas-Mess-Raum mit beispielsweise ca. 250$mm^3$, und eine gebotene Funktionalität zur Messung von Druck, Temperatur und der $CO_2$ Konzentration. Die Messung von Druck und Temperatur erfolgt beispielsweise im $CO_2$ Modul (z.B. im Gas-Sensor), wobei eine genaue Drift-Korrektur (beispielsweises einer $CO_2$-Konzentrations-Messung) ermöglicht sein kann. Eine Messung des Atemwegdrucks kann beispielsweise durch Differenzbildung zwischen den beiden barometrischen Drucksensoren (im Modul und im Gerät) erfolgen.

Zu den weiteren Vorteilen zählen beispielsweise:

- Eine sehr kleine Gas-Messkammer,
- Ein schneller Gas-Austausch über Diffusion über Viren-Filter,
- Es wird wenig Atem-Gas für die Analyse, geringes Totraumvolumen benötigt,
- Eine unmittelbare $CO_2$ Konzentration wird direkt am Mundstück, im Trachealtubus gemessen,
- Eine schelle Diagnose des metabolischen Patienten-Zustandes,
- Eine Messung direkt am Tubus,
- Eine Messung durch Bakterien/Viren-Filter,
- Keine Zeitverzögerung: atemzugaufgelöste Konzentrationsbestimmung der $CO_2$ Konzentration,
- Ein niedriger Energiebedarf, nicht verbrauchend, physikalisches Messprinzip,
- Eine Auto-Kalibrierung am Ende der Inspirations-Phase auf Frischluft-Konzentration.
- Eine mechanisch und fluidisch zuverlässige einfach und schnell wechselbare Vorrichtung über Rastnasen zum Einspannen des Fluss-Tubus in das wiederverwendbare Gerät.

Im Folgenden ist ein weiteres Ausführungsbeispiel gemäß der vorliegenden Erfindung gezeigt und beschrieben.

Fig. 7 zeigt einen Gasfluss während einer Inspiration (Einatmungsphase): Eine Spülung der kleinen Kammer beinhaltend das Sensor-Modul mit frischer Luft oder spezielle Gase (für Anästhesie als Beispiel), die als Kalibriergase verwendet werden können. Der Gasaustausch erfolgt hier durch ein Viren-Filter.

Fig. 8 zeigt einen Gasfluss während einer Exspiration (Ausatmungsphase): Hier kann die $CO_2$ Konzentration in der ausgeatmeten Luft der Patienten ausgewertet werden. Für die Diffusion der $CO_2$ Moleküle durch das Bakterien-Filter wird als Beispiel: mit 1$\mu$m Maschenweite eine Diffusionszeit von 7,2ms für einen Konzentrationssprung auf 5 vol% am Sensor benötigt.

Zu den Eigenschaften eines solchen Ausführungsbeispiels zählen beispielsweise:

- Ein Wegwerf-Fluss-Tubus (Disposable Flow-Tubus) mit Virenfilter,
- Eine Auskopplung des Messgases über Diffusion,
- Ein Leckage-freier Anschluss über O-Ringe und/oder Flächendichtung und/oder angespritzte 2K-Kunststoff-Dichtung,
- Eine sichere Vorrichtung über Rastnasen,
- Ein $CO_2$ Modul befindet sich im wiederverwendbaren Gerät.

Folgendes Ausführungsbeispiels zeigt beispielsweise einen Aufbau eines thermischen Gas-Sensors und ein entsprechendes Sensor-Prinzip.

Der thermische Gassensor aus den Fig. 9 und Fig. 10 besteht aus (oder umfasst) beispielsweise drei zwischen einem Rahmen freitragend gespannten filigranen kristallinen Silizium-Drähten, die vom zu analysierenden Gas umgeben sind. Fig. 10 zeigt einen Sensorchip mit Silizium-Mikrodrähten zur Unterscheidung von Gasgemischen. Der mittlere Draht wird mit einem beispielsweise periodischen Heizsignal beaufschlagt, die beiden zum Heizer links und rechts unsymmetrisch angeordneten Silizium-Drähte arbeiten als Temperatur-Sensoren. Der Wärmeübertrag erfolgt über die unbekannten Wärmeübergänge vom Heizer ins zu analysierende Gas und vom Gas in den Sensor-Draht. Durch die Messung der Temperatur-Antwort mit zwei identischen Sensoren in unterschiedlichen Abständen zum Heizer lassen sich die unbekannten Wärmeübergänge in der Messanordnung eliminieren. Phase und Amplitude der beiden Sensorsignale sind im Wesentlichen von der Wärmeübertragung durch das Gas abhängig.

In Fig. 11 ist das Grundprinzip des thermischen Sensors schematisch dargestellt: Deutlich zu sehen ist die räumliche Trennung von Heizer und Sensordrähten mit thermischer Kopplung durch das zu analysierende Gasgemisch, sowie die Messung mit Sensordrähten in unterschiedlichen Abständen zum Heizer.

Heizer und Sensor(en) sind im Medium getrennt angeordnet und sind vom zu analysierenden Gas umgeben. Der Wärmestrom vom Heizer zu den Temperatursensoren findet nur über das Gas selbst statt. Der Wärmetransport findet auch über die unbekannten Wärmeübergänge vom Heizer ins zu analysierende Gas und vom Gas in den Sensordraht statt. Bei einer Messung in zwei Abständen sind die Wärmeübergänge nahezu dieselben. Die Differenz beider Sensorsignale hängt im Wesentlichen von der Wärmeübertragung durch das Medium selbst ab.

Elektrische Analogie: Um die Wärmeströme zu identifizieren und abzuschätzen, wurde eine elektrische Analogie erstellt. Die Optimierung des Wärmeverlusts ist ein wesentlicher Faktor, um die Empfindlichkeit des Sensors zu steigern, ohne eine zu hohe Heizleistung einspeisen zu müssen.

Fig. 12 zeigt eine schematische Darstellung des Wärmetransports am Sensor. Der Wärmetransport vom Heizer (Temperatur TH) zum Sensor (Temperatur TS) findet im Wesentlichen durch das zu messenden Gas statt.

Bei einer sinusförmigen Heizleistung ergibt sich ein sinusförmiger Verlauf der Sensorsignale, der stark von den thermischen Eigenschaften des Gases abhängig ist, welches die Sensordrähte umgibt. Durch die Messung der Temperatur des Heizers mit zwei identischen Sensoren in unterschiedlichen Abständen zum Heizer lassen sich die unbekannten Wärmeübergänge in der Messanordnung eliminieren, wie schon o.a. beschrieben.

Zur Auswertung werden wie in Fig. 13 dargestellt, ausgesendete und empfangene Sinuswellen verglichen. Mit einer Kalibration des Signals über die Phasenverschiebung zwischen Heizer und den Sensoren kann beispielsweise der $CO_2$-Gehalt in Luft mit 0,2 vol% aufgelöst werden. Da Gase kompressibel sind und durch Druck- und Temperatur ihre Dichte ändern, sollten die entsprechenden Driften kompensiert werden.

Über die Auswertung weiterer Messgrößen, die der Sensor liefert, lassen sich Wärmeleitfähigkeit, Temperaturleitfähigkeit und bei bekannter Dichte des Gases auch die spezifische Wärmekapazität bestimmen - ein möglicher Weg, um auch unbekannte Gasgemische zu analysieren.

Fig. 13 zeigt weiterhin Signale bei einer Anregung mit einer sinusförmigen Heizleistung für $CO_2$ und $N_2$ im Vergleich. Bei gleicher Heizleistung unterscheiden sich die empfangenen Sensorsignale in Amplitude, Offset und Phasenlage.

Durch den konstruktiven Unterschied freitragender Brückenstrukturen gegenüber geschlossener Dünnschicht-Membranen wird weitgehend die parasitäre thermische Entkopplung zwischen Heizer und Detektor-Elementen erreicht und die Signalqualität deutlich erhöht. Aufgrund der geringen thermischen Masse des Heizers ist es möglich, den Heizer mit Frequenzen bis zu 300 Hertz zu modulieren, da die Wärme schnell zu- und abgeführt werden kann.

Ein weiterer Erfindungsaspekt bezieht sich auf eine Aufteilung in ein wieder verwendbares Gerät und einen Atemkanal, der beispielsweise ein Einwegartikel sein kann. Fig. 14. zeigt eine solche Trennung zwischen wieder verwendbarem Gerät und Atemkanal als Einwegartikel. Im linken Bild ist der Atemkanal als Einwegartikel grün / links schraffiert dargestellt. Er besteht aus dem Atemstrom-Kanal mit Standard-Konus-Anschlüssen und enthält optional einen MEMS Durchflusssensor und ein Filter.

Das blau / rechts schraffiert dargestellte Teil ist ein auf den Atemkanal aufsteckbares Messgerät, und kann beispielsweise mehrfach wiederverwendet werden, weil es nicht mit dem Atemgas des Patienten in Berührung kommt. Der hellblaue / kreuzschraffierte Block in der Darstellung auf der rechten Seite von Fig. 14 zeigt schematisch den Gas-Messraum als Hohlraum im Sensor-Gehäuse mit einer Größe von ca. 250mm3. Der $CO_2$-Sensor, beispielsweise ein thermischer Gassensor, befindet sich in diesem Hohlraum und ist nach außen gegen Berührung mit einem Gitter geschützt.

Das optionale Viren-/Bakterien-Filter verhindert, dass der CO2-Sensor durch das Atem-gas des Patienten kontaminiert wird. Das Filter ist hier als Bestandteil des Atemkanals ein Einwegartikel. Der Leckage-freie Anschluss der Gas-Mess-Kammer des CO2-Sensors wird entweder durch O-Ringe, eine Flächendichtung oder über eine angespritzter 2K-Kunststoff-Dichtung erreicht, die entweder Teil des wieder verwendbaren Gerätes oder des Atemkanals sein kann.

Ein weiterer Aspekt der Erfindung bezieht sich auf die Abschätzung der Diffusionszeit bis zu einem Konzentrationsausgleich. Der $CO_2$-Sensor sollte, da er zum wieder verwendbaren Teil gehört, möglichst vor jeglicher Kontamination durch das Atemgas des Patienten geschützt werden. Aus diesem Grund trennt ein Viren-/Bakterien-Filter den kontaminierten Bereich im Atemkanal von dem nicht kontaminierten wiederverwendbaren Messgerät mit dem $CO_2$-Sensor. Das Filter ist beispielsweise Bestandteil des Atemkanals (dem Einwegartikel), da er durch das Atemgas des Patienten kontaminiert ist.

Fig. 15 zeigt in einer schematischen Darstellung ein Ausführungsbeispiel eines CO2 Sensors. Dieser weist folgende Merkmale auf:

- ein wiederverwendbares angeclipstes Gehäuse - resusable clipped housing
- einen Wegwerf-Kanal - disposable channel
- einen Flusssensor (gehört zum Wegwerf-Kanal) - flow sensor (belongs to disposable channel)
- Atem - Breath
- einen $CO_2$-Sensor - $CO_2$ sensor
- eine Sensorkammer - Sensor chamber
- ein Bakterien-Filter - bacterial filter
- Wegwerf bzw. wegwerfbar - dispoable
- wiederverwendbar - reusable

Das Filter verringert die Diffusionsgeschwindigkeit der CO2-Moleküle in die Gas-Mess-Kammer des Sensor-Gehäuses, wodurch die Ansprechzeit des Sensors erhöht wird. Deshalb muss die Diffusions-Zeit bis zum Ausgleich der Konzentration im Atemkanal und in der Gas-Mess-Kammer des Sensors in Bezug auf Filterdurchmesser und Porengröße geschätzt werden.

In Fig. 16 ist eine Berechnung unter vereinfachten statischen Randbedingungen bei einer Temperatur von 300 K, atmosphärischem Druck und ohne Berücksichtigung der Atemfeuchte dargestellt.

Zu den Einflussparametern auf die Diffusion nach dem Gesetz von Fick gehören:

Expired air = ausgeatmete Luft
External Air = äußere Luft
Channel = Kanal
Sensor's chamber = Sensorkammer
Filter=Filter
Porosity=Porösität
Sensor=Sensor
Diffusion of gases through the filter=Diffusion von Gasen durch das Filter

Für den Diffusionsfluss J definiert das Erste Fick'sche Gesetz folgenden Zusammenhang:

$$J = -D\frac{\partial\varphi}{\partial x}$$

$$J = -D\frac{(C_2 - C_1)}{\delta}$$

wobei D der Diffusionskoeffizient des Gases 1 ins Gas 2 ist (als konstant angenommen), C1 und C2 die Konzentrationen des Stoffes in den beiden Gase sind und $\delta$ das geometrische Verhältnis ist.

Für die Berechnung wurden folgende Parameter festgelegt: $CO_2$-Konzentration des Exspirationsgases C1 = 1,62 $mol\cdot m^{-3}$, CO2-Konzentration in der Frischluft C2 = 1,62·10-3 $mol\cdot m^{-3}$, Diffusionskoeffizient D = $14\cdot 10^{-6}\ m^2\cdot s^{-1}$ (Diffusionskoeffizient für $CO_2$ in Luft unter atmosphärischem Druck bei 300 K) und für die Filter-Membran eine Porosität von 0,5 bei einer Dicke $\delta$ = 10 $\mu$m und einer effektiven Membranfläche von S = $1{,}03\cdot 10^{-4}\ m^2$, wobei das Volumen der Sensorkammer (des Gas-Mess-Raumes) mit $V1=5{,}14823\cdot 10^{-7} m^3$ bei einem ersten Prototyp und mit V2 = $1{,}378\cdot 10\text{-}7m^3$ beim derzeitigen Sensoraufbau angenommen wurde. Damit ergibt sich Diffusionsfluss von J = 2,25 mol·m-2·s-1, was bedeutet, dass pro Sekunde eine Stoffmenge von 2,25 Mol $CO_2$ eine 1 $m^2$ große Filterfläche durchqueren würde. Umgerechnet auf die tatsächliche Filterfläche ergibt sich ein Fluss von wenigen $\mu$mol/s der ausreicht, um die Gas-Mess-Kammer nach 7,2 ms (erster Prototyp) auf die Konzentration C1 des Exspirationsgases zu bringen.

Ist die Gas-Mess-Kammer mit V2 um Faktor 30 kleiner gegenüber V1, so ergibt sich eine Verzögerungszeit bis zum Konzentrationsausgleich von nur etwa 0,2 ms. Der zeitliche Diffusionsverlauf in Fig. 17 zeigt, dass je kleiner die Gas-Mess-Kammer gewählt wird, desto schneller der Konzentrationsausgleich am Sensor erfolgt.

Das Ergebnis der Abschätzung ist unterbestimmt, da sich die tatsächliche Diffusionszeit bis zum Konzentrationsausgleich aufgrund der Druckerhöhung während der Exspiration im Atemkanal entsprechend der Wrobleski Gleichung weiter verkürzt:

$$J = P\frac{(p_2 - p_1)}{\partial x}$$

wobei p1 der Druck im Atemkanal ist, p2 der Druck in der Gas-Mess-Kammer und P die Permeabilität des Filters. Bei einer Gasströmung im Kanal erhöht sich die Differenz p2 - p1, so dass der Diffusionsfluss zunimmt und die CO2-Diffusion durch das Filter ebenfalls zunimmt. Diese Druckdifferenz führt zu einer Strömung in die Sensorkammer hinein, die die Gasdiffusion durch das Filter begünstigt.

Strömungseinfluss auf das Sensorsignal : Das Signal des CO2-Sensors kann leicht gestört werden, da es gegenüber dem thermischen Strömungssignal eine geringere Sensitivität zeigt. Daher sollte der parasitäre Einfluss des Flusses auf das Signal des CO2-Sensors vermieden werden, um die Gaskonzentration genau messen zu können.

Filter, Geometrie des Einlaufgitters und miniaturisierte Gas-Mess-Kammer schaffen bei einem Ausführungsbeispiel ein beruhigtes Gebiet, in dem der thermische Sensor ungestört vom äußeren Durchfluss arbeiten kann.

Mögliche Anwendungsgebiete der Ausführungsbeispiele der Erfindung sind beispielsweise in der Medizintechnik für die Beatmung von Patienten (Kapnometrie) oder in der Erdgas Analyse, wo beispielsweise eine Bestimmung des Brennwertes eines Gases erfolgen soll. Für die Kapnometrie sind verschiedene $CO_2$ Sensoren im Einsatz, die nachfolgend zusammengefasst angeführt sind. Hauptsächlich wird mit der Infrarot-Spektroskopie die Absorption an den $CO_2$ Molekülen gemessen.

Fig. 18 zeigt ein Ausführungsbeispiel eines Metalloxid-Sensors (MOX).

Zu dessen Vorteile gehören folgende Merkmale:

- Einfaches Prinzip (Chemische Reaktion einer Dünnschicht)
- hohe Sensitivität bei kleinen Konzentrationen
- kostengünstig

Zu dessen Nachteile gehören folgende Merkmale:

- Sensor verbraucht sich
- keine Langzeitstabilität
- geringe Selektivität der Gasart
- für $CO_2$ nicht geeignet
- Betriebstemperatur bis zu 800 °C und dementsprechend Risiko für den Einsatz im Hauptstrom

Fig. 19 zeigt ein Ausführungsbeispiel eines Elektrochemisch potentiometrische Sensoren (NASICON).

Zu dessen Vorteile gehören folgende Merkmale:

- hohe Genauigkeit
- geringe Dimensionen

Zu dessen Nachteile gehören folgende Merkmale:

- Elektrodenmaterial verbraucht sich
- relativ teuer bei geringer Lebensdauer

Fig. 20 zeigt ein Ausführungsbeispiel eines nichtdispersiven Infrarot-Sensors (NDIR). Zu dessen Vorteile gehören folgende Merkmale:

- Infrarotabsorption bei 4,3 μm Wellenlänge, Messung der Konzentration von $CO_2$ im Atemgas durch Absorption
- nicht verbrauchend
- Messung in Hauptstrom möglich

Zu dessen Nachteile gehören folgende Merkmale:

- Die Funktionsweise bedingt einen entsprechend präzisen Aufbau teure Optik Komponenten
- sehr empfindliche Komponenten gegenüber Verschmutzung: Sekret und Atemfeuchte
- Alterung der Photodiode

Fig. 21 zeigt ein Ausführungsbeispiel eines MEMS Draht-Sensor (Hahn-Schickard)
Zu dessen Vorteile gehören folgende Merkmale:

- Geringer Bauraum
- Physikalisches Messprinzip - nicht verbrauchend
- Kostengünstig
- Messung im beruhigten Hauptstrom möglich

Zu dessen Nachteile gehören folgende Merkmale:

- Im Vorentwicklungsstadium - noch kein Produkt
- Messung der thermischen Gaseigenschaften Wärmeleitfähigkeit und Temperaturleitfähigkeit: keine echte Gasanalyse, Gas-Komponenten müssen bekannt sein
- Begrenzte thermische Auflösung (0,2% Vol. $CO_2$)

Für die Kapnometrie existieren verschiedene tragbare Geräte für die Atemgasanalytik. Beispielhaft kann ein Produkt der Firma Weinman Diagnostics genannt werden: Ein Tragbarer Kapnograph zur $CO_2$-Messung und -Auswertung, der intern mit einem Infrarotsensor arbeitet. Die Patientenanbindung erfolgt über eine Nasenbrille und die ausgeatmete Luft wird im Nebenstrom durch einen langen Schlauch zum Gerät zur Bestimmung des CO2 Gehaltes geführt.

Eine weiter Vorrichtung der Art ist das von Bluepoint medical angebotene CapnoTrue AMP, welches im Hauptstromverfahren mit dem IRMA Mainstream Analyzer von Phasein arbeitet.

Am Markt existieren heute verschiedene Systeme zur Patientenbeatmung. Diese werden unterschieden nach Einsatz im klinischen und im Homecare-Bereich (z.B. Systeme der Firmen Heinen+Löwenstein, Dräger und Stephan Medizintechnik). Die Systeme dieser Anbieter beinhalten nur in ihren Top-Varianten alle notwendigen Messeinrichtungen zur Bestimmung von Druck, Atemfluss und Atemgasanalyse. Dazu müssen mehrere Geräte kombiniert werden, die überwiegend patientenfern messen.

Die optionale Integration beider Sensoren ($CO_2$ und Strömung) in ein Sensorsystem kann zu einer deutlichen Reduzierung des Bauraums und des Systemgewichtes führen (ein wesentliches Kriterium bei intubierten Patienten). Erst der patientennahe Messort unmittelbar an Maske oder Tubus - so nah wie möglich an den Atemwegen - ermöglicht eine ausreichend genaue Messung, um Einflüsse durch Schläuche, Bewegungen oder andere Störquellen zu vermeiden. Durch das thermische Messprinzip werden außerdem genauere Strömungsmessungen und eine schnelle Gasanalyse erwartet.

Im Folgenden werden weitere Ausführungsbeispiele und Aspekte der Erfindung beschrieben, die für sich genommen oder in Verbindung mit weiteren hierin beschriebenen Aspekten bzw. Ausführungsbeispielen oder Merkmalen eingesetzt werden können.

Ein Ausführungsbeispiel (Aspekt 1) bezieht sich auf ein miniaturisiertes Gehäuse (Package) zur Konzentrationsbestimmung von Gasen, beispielsweise zur Bestimmung des $CO_2$ Anteils im Exspirationsgas bei der Ausatmung, welches ausgeführt ist als gasdichte Messkammer mit einer nach außen gegen Leckage abgedichteter Öffnung zum Gasaustausch über Diffusion, welches ausgeführt ist als Stapel bestehend aus

- einem Verdrahtungsträger,
- einem barometrischen MEMS Drucksensor,

  vorzugsweise (aber nicht notwendigerweise) einem barometrischen Höhenmesser mit digitaler Schnittstelle
  und (optional) hoher A/D Auflösung (vorzugsweise 24bit)

- und einem thermischen MEMS Gas-Sensor,

  wobei (optional) Heizer und mindestens zwei Detektoren im Gasmessraum freitragend aufgespannt sind,
  wobei (optional) thermische Gasantwort, barometrischer Gasdruck und Gastemperatur unmittelbar auf engstem Raum an einem Messort gemessen werden und
  (optional) aufgrund der mechanischen Auftrennung zwischen Heizer und Detektoren der Wärmetransfer vom Heizer zu den Detektoren vorwiegend über das Messgas erfolgt,
  wobei (optional) die Detektoren seitlich vom Heizer in unterschiedlichen definierten Abständen zu diesem angeordnet sind,
  wobei (optional) der Heizer des thermischen Sensors mit einer periodischen Heizleistung (vorzugsweise 120Hz oder größer) beaufschlagt wird und (optional) an den Detektoren die gaskonzentrationsabhängige Amplitude und Phasenverschiebung gegenüber dem Heizsignal ermittelt wird,
  wobei (optional) die Detektor-Signale sowohl zueinander als auch zum Heizersignal absolut und differentiell verglichen werden,
  wobei (optional) zur Kalibration auf die gasartabhängige Gaskonzentration (vorzugsweise $CO_2$) eine Synthese aus Differenz- und Summen-Bildung der Signale genutzt wird,
  wobei (optional) zur druck- und temperatur-abhängigen Driftkorrektur die mit dem barometrischen Drucksensor im Gasmessraum ermittelten Werte für Absolutdruck und Temperatur vorzugsweise über Polynome verrechnet wird, wobei (optional) die Temperurauflösung und absolute Genauigkeit über eine Temperatur-Kalibration der Detektoren des thermischen Gassensors und Nutzung deren Messsignale wesentlich erhöht werden kann,

- wobei (optional) das Sensor-Gehäuse ein Einlaufgitter als mechanischen Schutz aufweist,
- wobei (optional) das miniaturisierte Sensor-Package (Sensor-Gehäuse) zur Konzentrationsbestimmung von Gasen unmittelbar hinter einer Seitenbohrung am Strömungsrohr angeordnet ist,
- wobei (optional) das Messgas über Diffusion durch ein Bakterien- oder Virenfilter aus dem Strömungskanal ausgekoppelt wird und
- wobei (optional) der Konzentrationsausgleich unter normaler Atmung innerhalb von 10ms erfolgt, da aufgrund des miniaturisierten Aufbaus das Volumen des Gasmessraums bevorzugt kleiner als $250mm^3$ ist,
- wobei (optional) das miniaturisierte Sensor-Package (Sensor-Gehäuse) an das Strömungsrohr über einen elastischen O-Ring, eine Flachdichtung oder über angespritzen 2K Kunststoff vom Gehäuse mechanisch gegen Leckage gedichtet wird,
- wobei (optional) Filter, Geometrie des Einlaufgitters und miniaturisierte Gas-Mess-Kammer ein beruhigtes Gebiet schaffen, in dem der thermische Sensor ungestört vom äußeren Durchfluss arbeiten kann,
- wobei (optional) das Strömungsrohr welches das Bakterien- oder Virenfilter trägt als Wegwerfartikel ("Disposable") ausgeführt sein kann,
- wobei (optional) das miniaturisierte Sensor-Package (Sensor-Gehäuse) zur Konzentrationsbestimmung von Gasen einschließlich seiner Mikro-Prozessor basierten Signalauswertung nur wenige Gramm wiegt, eine geringe Baugröße besitzt und aufgrund der Verwendung von MEMS Bauteilen einen geringeren Leistungsbedarf bevorzugt kleiner 50mW aufweist und damit in Maske, Mundstück oder Trachealtubus unmittelbar am Patienten eingesetzt werden kann, um eine genaue und zeitlich unverfälschte Messung zu ermöglichen.

Ein weiteres Ausführungsbeispiel bezieht sich auf eine Vorrichtung nach Aspekt 1 in Kombination mit einem zweiten barometrischen Höhenmesser, der sich außerhalb des Strömungskanals im Messgerät befindet und den barometrischen Luftdruck des Raumes erfasst,

- wobei das Messgerät beispielsweise die Differenz aus dem vom barometrischen MEMS Drucksensor im miniaturisierten Sensor-Package (Sensor-Gehäuse) ermittelten Absolutdruck des Strömungskanals und dem barometrischen Luftdruck des Raumes bildet und damit den Atemdruck errechnet.

Ein weiteres Ausführungsbeispiel (Aspekt 3) bezieht sich auf eine Vorrichtung nach Aspekt1 oder Aspekt 2 in Kombination mit einem MEMS Strömungssensor im Strömungsrohr zur Messung der Atemwerte im Hauptstromverfahren,

- wobei beispielsweise mit Hilfe der vom barometrischen MEMS Drucksensor im miniaturisierten Sensor-Package ermittelten Werte für Absolutdruck und Gastemperatur der Atemluft aus dem vom MEMS Strömungssensor gemessenen Massenstrom in den aktuellen Volumenstrom unter ATP-Bedingungen (Ambient Temperature and Pressure=Umgebungstemperatur und Druck) umgerechnet wird.

Ein weiteres Ausführungsbeispiel (Aspekt 4) bezieht sich auf eine Vorrichtung gemäß einem der Aspekte 1 bis 3, ergänzt um einen Null-Punkt-Abgleich in Verbindung mit dem Fluss-Signal des Strömungs-Sensors im Atemtubus: Dynamische Kalibration des CO2-Sensors auf Frischgas bzw. Anästhesie-Gas-Konzentrationen aus dem Respirator.

Der thermische Sensor besitzt eine erhöhte Signal-Abhängigkeit gegenüber den Umwelteinflüssen Absolutdruck und Temperatur. D.h., werden diese Parameter nicht gemessen, dann würden falsche Konzentrationswerte für das $CO_2$ / oder andere Gase angenommen werden: Deshalb werden direkt am selben Messort (Stack) der Absolutdrucksensor plaziert, der neben dem barometrischen Luftdruck gleichzeitig auch die Temperatur misst.

Nun besteht im Einsatz die Möglichkeit, dass sich trotz Membran (Virenfilter) über eine längere Zeitdauer leichte Verschmutzungen an den Detektordrähten des Sensors absetzen können, wobei das Signal driften kann. Auch die Elektronik zeigt eine umweltabhängige (meist Temperatur) Drift.

Deshalb kann es praktisch sinnvoll sein, den Sensor in Gegenwart eines bekannten Gases auf seinen Null-Punkt abzugleichen. Sehr oft wird dazu Frischluft also die Raumluft verwendet. Das ist ein vorteilhaftes Vorgehen beim Einschalten / Inbetriebnahme des Sensors an einem neuen Patienten oder nach Sensor-Wechsel. Der Sensor wird aus seiner Verpackung genommen, elektrisch angeschlossen und der Frischluft beim Initialisieren ausgesetzt. Bei bekannten Luftbedingungen (Standard) kalibriert er sich selbst. Dieses Vorgehen ist sowohl bei Hitzdrahtanemometern (Atemgasmessung) als auch bei Kapnometern (NDIR $CO_2$-Messung) üblich.

Vorstellbar ist (also optionales Merkmal bei Ausführungsbeispielen der Erfindung) eine dynamische Nachkorrektur während der Beatmung: Wenn der angeschlossene Strömungstubus das Ende der Inspirationsphase detektiert (dem Patienten wurde soeben Frischluft oder mit Anästhesiegas angereicherte Frischluft zugeführt), könnte der aktuelle Messwert des Sensors als Nullpunkt für diesen bekannten Gaszustand interpretiert und der Sensor ggf. nachjustiert werden. (Die Anästhesie-Gaskonzentration aus dem Respirator ist dem Respirator beispielsweise bekannt und wird dem Spirometer-Device (Spirometer-Bauteil) mitgeteilt)

Aber auch ein umgekehrtes Szenario wäre denkbar: Durch Fehler im Patienten-Schlauchsystem wird das ausgeatmete $CO_2$ nicht korrekt abgeführt und die $CO_2$-Konzentration (auch bei der Frischluft) steigt kritisch an: Hier dürfte nicht dynamisch nachkalibriert werden, sondern es muss aufgrund der zu hohen $CO_2$-Konzentration bei der Inspiration (Frischluft-Messung) ein Alarm ausgelöst werden.

Aufgrund der unmittelbaren geometrischen Nähe der Messorte für Strömung und GasKonzentration laufen Flow-Signal (Fluss-Signal) und CO2-Signal bei unserem System synchron. So lassen sich Fehler oder Nachjustierungen direkt innerhalb eines Atemhubes korrigieren.

Im noch üblichen Seitenstromverfahren der Kapnometrie wird Gas aus dem Hauptstrom aktiv abgesaugt und gelangt in den dünnen Schläuchen über ca. 1,5m an das Kapnometer im Respirator. Dadurch entsteht ein zeitlicher Versatz zwischen Durchfluss- und $CO_2$-Signal, der softwareseitig/rechnerisch korrigiert wird. Nur Kapnometer mit direkter Durchstrahlung des Atemstromtubus (optische Fenster) messen zeitsynchron zum Flow-Signal (Fluss-Signal).

Gemäß einem weiteren Aspekt der Erfindung erfolgt eine Auskopplung in eine abgeschlossenen Kammer und Gasdiffusion durch Filter.

Gemäß einem weiteren Aspekt der Erfindung weisen Ausführungsbeispiele ein geringes Kammer-Gasvolumen auf, was besonders vorteilhaft ist. Aufgrund dessen wird es erst möglich, über Diffusion ein konzentrationsabhängiges dynamisches Signal am Sensor synchron zum Atemzyklus darzustellen.

Gemäß einem Aspekt sind eine oder mehrere der folgenden Vorgaben für Membran und Kammer (bzw. für den Sensor oder die Sensoranordnung allgemein) zu beachten:

- Die Membran vor der Sensor-Messkammer teilt den Gasraum in eine beruhigte Zone ab: Die nahezu Strömungsfreie Zone ist eine wichtige Voraussetzung für die thermische Gaskonzentrations-Messung.

- Wichtig ist bei einigen Ausführungsbeispielen, dass das sehr geringe Kammervolumen in Verbindung mit der kleinen Diffusionszeitkonstante erst die dynamische Konzentrationsmessung zulässt. Schließlich ist der Gasaustausch in der Messkammer ein passiver Prozess und abhängig von der mittleren freien Weglänge der Teilchen. (siehe theoretische Diskussion der Diffusions-Zeiten weiter oben)

- Die Membran ist (optional) eine hydrophobe Viren- / oder Bakterien-Membran, die z.B. vom Millipore zugekauft wird. Das optionale Filter kann beispielsweise das Eindringen von Flüssigkeiten in die Messkammer verhindern.

- Der Filtermembrandurchmesser sollte den Durchmesser des Atemstromtubus nicht überschreiten, sonst müsste die Filterdichtung über die Kontur/Flanken des Tubus ausgeführt werden und wäre nicht mehr eine ebene Fläche.

Dadurch müsste der Anpressdruck zwischen Tubus und Gerät erhöht werden, da die Kammer gasdicht (Leckagefrei) an den Atemtubus angeschlossen werden sollte. Eine gute Wahl sind beispielsweise 60% des äußeren Atemtubus-Rohrdurchmessers.

- Da das Filter bei Gebrauch verschmutzt, ist es (optional) Teil des Einweg-Atemtubus: Somit werden bei neuem Tubus definierte Diffusionskonstanten erreicht. Der Gas-Sensor, der im wiederverwendbaren Gerät platziert ist, trägt selbst ein Gitter als mechanischen Schutz, damit sich das Filter nicht unkontrolliert in den Messraum durchbiegt und das Ergebnis verfälscht (mech. Abstützen der Filter-Membran bei Überdruck / Husten, Verhindern des unkontrollierten Reißens). Das Sensor-Gitter kann selbst auch eine Filter-Membran tragen, die bei der Nass-Sterilisation des Gerätes das Eindringen von Desinfektionslösung in den Messraum verhindert. (Diese zusätzliche Membran muss beim Gasaustausch / Dimensionierung berücksichtigt werden.)

- Die Dichtung kann entweder an der Geräteseite um den Sensor erfolgen (heutige Lösung mit einer O-Ring-Dichtung) oder durch eine an den Wegwerf-Tubus angespritzte / mit dem Filter eingeklebte Dichtlippe oder beides.

- Der mechanische (Steck-)Verschluss des Atemstrumtubus und das Gerät sollte die Leckagefreiheit zwischen Tubus und Messkammer gewähren.

- Die Membran dient (optional) der Trennung vor bakterieller / vireller Kontamination des wieder verwendbaren Gerätes aus dem Atemgas, schützt aber auch gleichzeitig den Sensor vor Eindringen von Flüssigkeiten (niederschlagende Feuchte, Sputum)

- Die Membran dient allgemein als Bakterien-/Viren-Filter

- das Messvolumen ist bei Ausführungsbeispielen relativ klein

- der Sensor insgesamt ist ebenso relativ klein

Gemäß einem Aspekt ist nicht nur ein mittlerer Wert der $CO_2$-Konzentration messbar, sondern eine dynamische Änderung wie diese in der Atemgasanalytik gefordert wird.

Gemäß einem Aspekt handelt es sich bei dem Drucksensor beispielsweise um einen Sensor vom Typ LPS25H vom Hersteller STMicroelectronics, wie in Fig. 22 gezeigt. Hier wird in Draufsicht ein R1 Druck-Anschluss (pressure port) und eine Markierung Pin1 vom Anschluss 1 gezeigt. Es kann aber auch ein beliebiger barometrischer Drucksensor zum Einsatz kommen, dessen geometrische Abmessungen geeignet sind für den Aufbau der Sensoranordnung und dessen Messfrequenz, Messauflösung und Genauigkeit im geforderten Bereich liegt.

Die Fig. 23 und 24 zeigen weitere Ausführungsbeispiele und optionale Merkmale, wie z. B.:

- Trennung in wiederverwendbares Gerät und Atemkanal als Wegwerfprodukt zur Einmalverwendung (zum Beispiel Tubus und Flussensor als Einweg-Teile)
- Messung von Flow, Druck, Temperatur und $CO_2$
- Umgang mit Atemdynamik und Feuchte
- Kommunikation über Bluetooth oder USB Stream

Die Fig. 25 und 26 zeigen weitere Ausführungsbeispiele mit Bezug auf eine erfinderische Systemarchitektur (Details optional): Der Flow-Sensor bezieht sich hier auf einen FlussSensor.

Die Fig. 27 und 28 zeigen weitere Ausführungsbeispiele mit Bezug auf weitere beispielhafte Vorrichtungen in einem modularen Aufbau.

tube=Rohr; $CO_2$Module=$CO_2$-Modul; plastic parts=Plastikteile;
Flow Module = Fluss-Modul; tube with flow sensor=Rohr mit Flußsensor

Fig. 29 zeigt beispielhaft einen Einweg-Tubus einer Multisensor-Plattform zur patientennahen Messung von $CO_2$-Konzentration und Volumenstrom der Atemluft.

Fig. 30 zeigt ein Ausführungsbeispiele eines wiederverwendbares Geräts, das in Fig. 31 auf einem Einweg-Tubus einer Multisensor-Plattform zur patientennahen Messung von $CO_2$-Konzentration und Volumenstrom der Atemluft aufgekoppelt ist.

## Patentansprüche

**1.** Sensoranordnung (100), mit folgenden Merkmalen:

einem barometrischen Drucksensor (10); und einem thermischen Gassensor (20);
**dadurch gekennzeichnet, dass** der thermische Gassensor (20) derart auf dem barometrischen Drucksensor (10) angeordnet ist, dass eine für Gase durchlässige Messstruktur (22) des thermischen Gassensors (20) vor einer Gas-Einlassöffnung des barometrischen Drucksensors (10) oder vor einer druckempfindlichen Oberfläche des barometrischen Drucksensors (10) angeordnet ist;
wobei der thermische Gassensor (20) einen Rahmen aufweist, der auf dem barometrischen Drucksensor (10) angeordnet ist, und

wobei der Rahmen die für Gase durchlässige Messstruktur (22) trägt, so dass aktive Bereiche der für Gase durchlässigen Messstruktur (22) einen freien Innenbereich des thermischen Gassensors (20), der von dem Rahmen umgeben ist, überspannen;
wobei die Gas-Einlassöffnung des barometrischen Drucksensors (10) oder die druckempfindliche Oberfläche des barometrischen Drucksensors (10) an den freien Innenbereich des thermischen Gassensors (20) angrenzt.

**2.** Sensoranordnung gemäß Anspruch 1, wobei der thermische Gassensor (20) mindestens drei elektrische Leiterstrukturen (30, 32, 34) umfasst, wobei die elektrischen Leiterstrukturen (30, 32, 34) durch Zwischenräume beabstandet sind, wobei eine erste elektrische Leiterstruktur eingerichtet ist, um mit einem Heizsignal beaufschlagt zu werden und wobei eine zweite elektrische Leiterstruktur und eine dritte elektrische Leiterstruktur bezüglich der ersten elektrischen Leiterstruktur unterschiedlich beabstandet angeordnet sind, und wobei die zweite elektrische Leiterstruktur und die dritte elektrische Leiterstruktur eingerichtet sind, um als Temperatursensoren zu arbeiten.

**3.** Sensoranordnung gemäß Anspruch 2, wobei die elektrischen Leiterstrukturen (30, 32, 34) kristalline Siliziumdrähte sind oder wobei die elektrischen Leiterstrukturen (30, 32, 34) ein polykristalliner Heizer auf Membranmaterial sowie Halbleiter Temperatur-Detektoren oder Thermostapel sind.

**4.** Sensoranordnung gemäß Anspruch 1, wobei der thermische Gassensor (20) mindestens zwei elektrische Leiterstrukturen (30, 32) umfasst, wobei die elektrischen Leiterstrukturen (30, 32) durch zumindest einen Zwischenraum beabstandet sind, wobei eine erste elektrische Leiterstruktur eingerichtet ist, um mit einem Heizsignal beaufschlagt zu werden und wobei eine zweite elektrische Leiterstruktur eingerichtet ist, um als Temperatursensor zu arbeiten.

**5.** Sensoranordnung gemäß Anspruch 4, wobei die Sensoranordnung ausgelegt ist, um in einem ersten Zeitintervall die erste Leiterstruktur mit einem Heizsignal zu beaufschlagen und die zweite Leiterstruktur als Temperatursensor zu verwenden, und
wobei die Sensoranordnung ausgelegt ist, um in einem zweiten Zeitintervall die zweite Leiterstruktur mit einem Heizsignal zu beaufschlagen und die erste Leiterstruktur als Temperatursensor zu verwenden.

**6.** Sensoranordnung gemäß Anspruch 1, wobei der thermische Gassensor (20) mindestens drei elektrisch leitfähige Stege umfasst, wobei die Stege durch Zwischenräume beabstandet sind, wobei eine Metallisierung oder Dotierung eines ersten Stegs eingerichtet ist, um mit einem Heizsignal beaufschlagt zu werden und wobei ein zweiter und ein dritter Steg unsymmetrisch bezüglich des ersten Stegs angeordnet sind, und wobei Metallisierungen oder Dotierungen des zweiten Stegs und des dritten Stegs eingerichtet sind, um als Temperatursensoren zu arbeiten.

**7.** Sensoranordnung gemäß einem der Ansprüche 2 bis 6, wobei die elektrischen Leiterstrukturen (30, 32, 34) oder Drähte oder Stege eingerichtet sind, um von einem zu analysierenden Gas umgeben zu sein, und wobei die erste elektrische Leiterstruktur oder der erste Draht oder der erste Steg eingerichtet ist, um einen Wärmeübertrag über das zu analysierende Gas auf die zweite elektrische Leiterstruktur oder auf den zweiten Draht oder auf den zweiten Steg und auf die dritte elektrische Leiterstruktur oder auf den dritten Draht oder auf den dritten Steg zu ermöglichen, und wobei die zweiten und dritten elektrischen Leiterstrukturen (30, 32, 34) oder Drähte oder Stege eingerichtet sind, um als Sensoren für den Wärmeübertrag zu dienen.

**8.** Sensoranordnung gemäß einem der Ansprüche 1 bis 7, wobei der thermische Gas-sensor (20) ein Trägermaterial aufweist,

wobei der thermische Gassensor (20) in einem zentralen Bereich eine durchgehende Ausnehmung aufweist, die sich von einer dem barometrischen Drucksensor (10) abgewandten Oberfläche des thermischen Gassensors (20) bis hin zu einer dem barometrischen Drucksensor (10) zugewandten Oberfläche des thermischen Gassensors (20) erstreckt, und
wobei die für Gase durchlässige Messstruktur (22) in einem Bereich der Ausnehmung angeordnet ist.

**9.** Sensoranordnung gemäß einem der Ansprüche 1 bis 8, wobei der thermische Gas-sensor (20) mittels eines Klebstoffs mit dem barometrischen Drucksensor (10) verbunden ist, so dass der Klebstoff nicht mit der Gas-Einlassöffnung des barometrischen Drucksensors (10) oder mit der druckempfindlichen Oberfläche des barometrischen Drucksensors (10) in Kontakt ist.

**10.** Sensoranordnung gemäß einem der Ansprüche 1 bis 9, wobei die Sensoranordnung ein Leiterplattenmaterial aufweist;

wobei auf einer Seite des Leiterplattenmaterials der barometrische Drucksensor (10) und auf diesem der thermische Gassensor (20) angeordnet sind, und wobei auf einer anderen, von dem Drucksensor und dem thermischen Gassensor (20) abgewandten Seite des Leiterplattenmaterials ein Stecker oder Lötkontakt zur elektrischen Kontaktierung angeordnet ist, oder wobei der barometrische Drucksensor (10) und auf diesem der thermische Gassensor (20) in einer Ausnehmung des Leiterplattenmaterials angeordnet sind, und wobei auf einer Seite des Leiterplattenmaterials ein Stecker zur elektrischen Kontaktierung angeordnet ist.

**11.** Sensoranordnung gemäß einem der Ansprüche 1 bis 10, wobei die Sensoranordnung eine Auswerteeinrichtung umfasst, wobei die Auswerteeinrichtung eingerichtet ist, um eine Gaskonzentration basierend auf einer Phase und einer Amplitude von Sensorsignalen, die unter Verwendung des Gassensors erhalten werden, und abhängig von einer von dem barometrischen Drucksensor (10) gelieferten Druckinformation und ggf. Temperaturinformation zu ermitteln.

**12.** Sensoranordnung gemäß einem der Ansprüche 1 bis 11, wobei die Sensoranordnung von einem Gehäuse umgeben ist, das in seinem Inneren ein Volumen bereitstellt, in dem sich die Sensoranordnung befindet,

wobei das Gehäuse eine Gehäuseöffnung aufweist, durch die ein zu analysierendes Gas von einer Außenseite des Gehäuses zu der sich in dem Volumen befindlichen Sensoranordnung durch einen Diffusionsvorgang gelangen kann, wobei die Öffnung des Gehäuses eine Membran aufweist, die eingerichtet ist, um die Sensoranordnung vor einer Verschmutzung zu schützen und wobei die Membran eigerichtet ist, um eine Diffusion eines zu analysierenden Gases zu ermöglichen.

**13.** Sensorvorrichtung, mit folgenden Merkmalen:

einem Strömungskanal (700),
wobei der Strömungskanal (700) in einer Wandung eine Öffnung aufweist; und
einer Sensoranordnung gemäß einem der Ansprüche 1 bis 12, wobei die Sensoranordnung (100) derart angeordnet ist, dass die Sensoranordnung durch die Öffnung mit dem Inneren des Strömungskanals (700) räumlich verbunden ist, um einen Gasaustausch zwischen dem Inneren des Strömungskanals (700) und der Sensoranordnung zu ermöglichen.

**14.** Sensorvorrichtung gemäß Anspruch 13, wobei die Sensorvorrichtung so ausgelegt ist, dass der Rahmen einen Gas-Messraum der Gehäuseöffnung nach außen abdichtet, wobei eine Zeitdauer, bis zum Ausgleich der Gaskonzentration im Bereich des Gassensors um höchstens 0,5 vol% von der Gaskonzentration im Strömungskanal (700) abweicht, kleiner als 10 ms ist.

**15.** Sensorvorrichtung gemäß einem der Ansprüche 13 oder 14, wobei die Sensorvorrichtung so ausgelegt ist, dass eine die Sensoranordnung enthaltende Kammer ein Strömungsberuhigtes Gebiet darstellt.

**16.** Sensorvorrichtung gemäß einem der Ansprüche 13 bis 15, wobei die Sensorvorrichtung einen Strömungssensor (706) aufweist, wobei der Strömungssensor (706) derart angeordnet ist, um eine Strömungsgeschwindigkeit, und/oder einen Gas-Massestrom und/oder einen Volumenstrom in dem Strömungskanal (700) ermitteln zu können.

**17.** Sensorvorrichtung gemäß einem der Ansprüche 13 bis 16, wobei die Sensorvorrichtung einen zweiten barometrischen Drucksensor aufweist, der ausgelegt ist, um einen Umgebungsdruck zu messen.

**18.** Sensorvorrichtung gemäß einem der Ansprüche 13 bis 17, wobei die Sensorvorrichtung ausgelegt ist, um basierend auf einer Information über eine Strömungsgeschwindigkeit von Frischluft oder mit Anästhesiegas angereicherte Frischluft in dem Strömungskanal (700) und/oder basierend auf einer Information über eine Strömungsrichtung von Frischluft oder mit Anästhesiegas angereicherte Frischluft in dem Strömungskanal (700) einen Zeitpunkt für eine Kalibrierung zu erkennen, und um ansprechend darauf eine Kalibrierung des thermischen Gassensors (20) durchzuführen.

## Claims

**1.** Sensor arrangement (100), comprising:

a barometric pressure sensor (10); and
a thermal gas sensor (20);
**characterized in that** the thermal gas sensor (20) is arranged on the barometric pressure sensor (10) such that a gas-permeable measurement structure (22) of the thermal gas sensor (20) is arranged in front of a gas inlet opening of the barometric pressure sensor (10) or in front of a pressure-sensitive surface of the barometric pressure sensor (10);
wherein the thermal gas sensor (20) comprises a frame that is arranged on the barometric pressure sensor (10), and
wherein the frame carries the gas-permeable measurement structure (22) such that active areas of the gas-permeable measurement structure (22) span a free inner area of the thermal gas sensor (20) surrounded by the frame; wherein the gas inlet opening of the barometric pressure sensor (10) or the pressure-sensitive surface of the barometric pressure sensor (10) borders on the free inner area of the thermal gas sensor (20).

**2.** Sensor arrangement according to claim 1, wherein the thermal gas sensor (20) includes at least three electric conductor structures (30, 32, 34), wherein the electric conductor structures (30, 32, 34) are spaced apart by gaps, wherein a first electric conductor structure is configured to be provided with a heating signal and wherein a second electric conductor structure and a third electric conductor structure are arranged so as to be spaced apart differently with respect to the first electric conductor structure, and wherein the second electric conductor structure and the third electric conductor structure are configured to operate as temperature sensors.

**3.** Sensor arrangement according to claim 2, wherein the electric conductor structures (30, 32, 34) are crystalline silicon wires or wherein the electric conductor structures (30, 32, 34) are a polycrystalline heater on a membrane material as well as semiconductor temperature detectors or thermostacks.

**4.** Sensor arrangement according to claim 1, wherein the thermal gas sensor (20) includes at least two electric conductor structures (30, 32), wherein the electric conductor structures (30, 32) are spaced apart by at least one gap, wherein a first electric conductor structure is configured to be provided with a heating signal and wherein a second electric conductor structure is configured to operate as temperature sensor.

**5.** Sensor arrangement according to claim 4, wherein the sensor arrangement is configured to provide the first conductor structure with a heating signal in a first time interval and to use the second conductor structure as temperature sensor, and wherein the sensor arrangement is configured to provide the second conductor structure with a heating signal in a second time interval and to use the first conductor structure as temperature sensor.

**6.** Sensor arrangement according to claim 1, wherein the thermal gas sensor (20) includes at least three electrically conductive ridges, wherein the ridges are spaced apart by gaps, wherein a metallization or doping of a first ridge is configured to be provided with the heating signal and wherein a second and a third ridge are arranged asymmetrically with respect to the first ridge and wherein metallizations or dopings of the second ridge and the third ridge are configured to operate as temperature sensors.

**7.** Sensor arrangement according to one of claims 2 to 6, wherein the electric conductor structures (30, 32, 34) or wires or ridges are configured to be surrounded by a gas to be analyzed and wherein the first electric conductor structure or the first wire or the first ridge are configured to allow heat transfer via the gas to be analyzed to the second electric conductor structure or to the second wire or to the second ridge and to the third electric conductor structure or to the third wire or to the third ridge, and wherein the second and third electric conductor structures (30, 32, 34) or wires or ridges are configured to serve as sensors for the heat transfer.

**8.** Sensor arrangement according to one of claims 1 to 7, wherein the thermal gas sensor (20) comprises a carrier material,

wherein the thermal gas sensor (20) comprises a continuous recess in a central area extending from a surface of the thermal gas sensor (20) facing away from the barometric pressure sensor (10) up to a surface of the thermal gas sensor (20) facing the barometric pressure sensor (10) and
wherein the gas-permeable measurement structure (22) is arranged in an area of the recess.

**9.** Sensor arrangement according to one of claims 1 to 8, wherein the thermal gas sensor (20) is connected to the barometric pressure sensor (10) by means of an adhesive, such that the adhesive is not in contact with the gas inlet opening of the barometric pressure sensor (10) or with the pressure-sensitive surface of the barometric pressure sensor (10).

**10.** Sensor arrangement according to one of claims 1 to 9, wherein the sensor arrangement comprises a printed circuit board material;

wherein the barometric pressure sensor (10) and on top of the same the thermal gas sensor (20) are arranged on one side of the printed circuit board material and wherein a plug or solder contact for electric contacting are arranged on another side of the printed circuit board material facing away from the pressure sensor and the thermal gas sensor (20), or
wherein the barometric pressure sensor (10) and on top of the same the thermal gas sensor (20) are arranged in a recess of the printed circuit board material and wherein a plug for electric contacting is arranged on a side of the printed circuit board material.

**11.** Sensor arrangement according to one claims 1 to 10, wherein the sensor arrangement includes evaluation means, wherein the evaluation means are configured to determine a gas concentration based on phase and amplitude of sensor signals obtained by using the gas sensor and in dependence on pressure information provided the barometric pressure sensor (10) and possibly temperature information.

**12.** Sensor arrangement according to one of claims 1 to 11, wherein the sensor arrangement is surrounded by a housing providing a volume within the same where the sensor arrangement resides,

wherein the housing comprises a housing opening through which a gas to be analyzed can reach the sensor arrangement within the volume from an outside of the housing by a diffusion process.
wherein the opening of the housing comprises a membrane that is configured to protect the sensor arrangement from contamination and wherein the membrane is configured to allow diffusion of a gas to be analyzed.

**13.** Sensor apparatus, comprising:

a flow channel (700),
wherein the flow channel (700) includes an opening in a wall; and
a sensor arrangement according to one of claims 1 to 12, wherein the sensor arrangement (100) is arranged such that the sensor arrangement is spatially connected to the inside of the flow channel (700) through the opening to allow gas exchange between the inside of the flow channel (700) and the sensor arrangement.

**14.** Sensor apparatus according to claim 13, wherein the sensor apparatus is configured such that the frame seals a gas-measuring space of the housing opening to the outside, wherein a time period up to a compensation of the gas concentration in the area of the gas sensor deviating by at most 0.5 vol% from the gas concentration in the flow channel (700) is less than 10 ms.

**15.** Sensor apparatus according to one of claims 13 or 14, wherein the sensor apparatus is configured such that a chamber included in the sensor arrangement represents an area with steadied flow.

**16.** Sensor apparatus according to one of claims 13 to 15, wherein a sensor apparatus comprises a flow sensor (706), wherein the flow sensor (706) is arranged to be able to determine a flow velocity and/or a gas mass flow and/or a volume flow in the flow channel (700).

**17.** Sensor apparatus according to one of claims 13 to 16, wherein the sensor apparatus comprises a second barometric pressure sensor that is configured to measure an environmental pressure.

**18.** Sensor apparatus according to one of claims 13 to 17, wherein the sensor apparatus is configured to detect, based on information on a flow velocity of fresh air or fresh air enriched with anesthetic gas in the flow channel (700) and/or based on information on a flow direction of fresh air or fresh air enriched with anesthetic gas in the flow channel (700), a time for calibration and to perform calibration of the thermal gas sensor (20) in response thereto.

## Revendications

**1.** Aménagement de capteurs (100), aux caractéristiques suivantes:

un capteur de pression barométrique (10); et
un capteur de gaz thermique (20);
**caractérisé par le fait que** le capteur de gaz thermique (20) est disposé sur le capteur de pression barométrique (10) de sorte qu'une structure de mesure perméable aux gaz (22) du capteur de gaz thermique (20) soit disposée devant une ouverture d'entrée de gaz du capteur de pression barométrique (10) ou devant une surface sensible à la pression du capteur de pression barométrique (10);
dans lequel le capteur de gaz thermique (20) présente un cadre qui est disposé sur le capteur de pression barométrique (10), et
dans lequel le cadre porte la structure de mesure perméable aux gaz (22), de sorte que les zones actives de la structure de mesure perméable aux gaz (22) enjambent une zone intérieure libre du capteur de gaz thermique (20) qui est entourée par le cadre;
dans lequel l'ouverture d'entrée de gaz du capteur de pression barométrique (10) ou la surface sensible à la pression du capteur de pression barométrique (10) est adjacente à la zone intérieure libre du capteur de gaz thermique (20).

**2.** Aménagement de capteurs selon la revendication 1, dans lequel le capteur de gaz thermique (20) comporte au moins trois structures conductrices électriques (30, 32, 34), dans lequel les structures conductrices électriques (30, 32, 34) sont espacées l'une de l'autre par des interstices, dans lequel une première structure conductrice électrique est conçue pour qu'il y soit appliqué un signal de chauffage et dans lequel une deuxième structure conductrice électrique et une troisième structure conductrice électrique sont disposées de manière espacée différente par rapport à la première structure conductrice électrique, et dans lequel la deuxième structure conductrice électrique et la troisième structure conductrice électrique sont conçues pour fonctionner comme des capteurs de température.

3. Aménagement de capteurs selon la revendication 2, dans lequel les structures conductrices électriques (30, 32, 34) sont des fils de silicium cristallins ou dans lequel les structures conductrices électriques (30, 32, 34) sont un élément chauffant poly-cristallin sur un matériau de membrane ainsi que des détecteurs de température à semi-conducteur ou des piles thermiques.

4. Aménagement de capteurs selon la revendication 1, dans lequel le capteur de gaz thermique (20) comporte au moins deux structures conductrices électriques (30, 32), dans lequel les structures conductrices électriques (30, 32) sont espacées par au moins un interstice, dans lequel une première structure conductrice électrique est conçue pour qu'il y soit appliqué un signal de chauffage et dans lequel une deuxième structure conductrice électrique est conçue pour fonctionner comme un capteur de température.

5. Aménagement de capteurs selon la revendication 4, dans lequel l'aménagement de capteurs est conçu pour appliquer, dans un premier intervalle de temps, un signal de chauffage à la première structure conductrice et pour utiliser la deuxième structure conductrice comme capteur de température, et
dans lequel l'aménagement de capteurs est conçu pour appliquer, dans un deuxième intervalle de temps, un signal de chauffage à la deuxième structure conductrice et pour utiliser la première structure conductrice comme capteur de température.

6. Aménagement de capteurs selon la revendication 1, dans lequel le capteur de gaz thermique (20) comporte au moins trois barres électriquement conductrices, dans lequel les barres sont espacées l'une de l'autre par des interstices, dans lequel une métallisation ou un dopage d'une première barre est conçu pour qu'il y soit appliqué un signal de chauffage et dans lequel une deuxième et une troisième barre sont disposées de manière asymétrique par rapport à la première barre, et dans lequel les métallisations ou dopages de la deuxième barre et de la troisième barre sont conçus pour fonctionner comme des capteurs de température.

7. Aménagement de capteurs selon l'une des revendications 2 à 6, dans lequel les structures conductrices électriques (30, 32, 34) ou les fils ou les barres sont conçus pour être entourés par un gaz à analyser, et dans lequel la première structure conductrice électrique ou le premier fil ou la première barre est conçu pour permettre un transfert de chaleur par l'intermédiaire du gaz à analyser vers la deuxième structure conductrice électrique ou vers le deuxième fil ou vers la deuxième barre et vers la troisième structure conductrice électrique ou vers le troisième fil ou vers la troisième barre, et dans lequel les deuxième et troisième structures conductrices électriques (30, 32, 34) ou fils ou barres sont conçus pour servir de capteurs pour le transfert de chaleur.

8. Aménagement de capteurs selon l'une des revendications 1 à 7, dans lequel le capteur de gaz thermique (20) présente un matériau porteur,
dans lequel le capteur de gaz thermique (20) présente, dans une zone centrale, un évidement continu qui s'étend d'une surface du capteur de gaz thermique (20) éloignée du capteur de pression barométrique (10) jusqu'à une surface du capteur de gaz thermique (20) orientée vers le capteur de pression barométrique (10), et dans lequel la structure de mesure perméable aux gaz (22) est disposée dans une zone de l'évidement.

9. Aménagement de capteurs selon l'une des revendications 1 à 8, dans lequel le capteur de gaz thermique (20) est connecté au capteur de pression barométrique (10) au moyen d'un adhésif, de sorte que l'adhésif ne soit pas en contact avec l'ouverture d'entrée de gaz du capteur de pression barométrique (10) ou avec la surface sensible à la pression du capteur de pression barométrique (10).

10. Aménagement de capteurs selon l'une des revendications 1 à 9, dans lequel l'aménagement de capteurs présente un matériau de carte de circuit imprimé;

dans lequel est disposé, sur une face du matériau de la carte de circuit imprimé, le capteur de pression barométrique (10) et, sur ce dernier, le capteur de gaz thermique (20), et sur une autre face du matériau de la carte de circuit imprimé, opposée au capteur de pression et au capteur de gaz thermique (20), une fiche ou un contact brasé pour l'amenée en contact électrique, ou
dans lequel le capteur de pression barométrique (10) et sur ce dernier le capteur de gaz thermique (20) sont disposés dans un évidement dans le matériau de carte de circuit imprimé, et dans lequel est disposée, sur une face du matériau de carte de circuit imprimé, une fiche pour l'amenée en contact électrique.

11. Aménagement de capteurs selon l'une des revendications 1 à 10, dans lequel l'aménagement de capteurs comporte un moyen d'évaluation, dans lequel le moyen d'évaluation est conçu pour déterminer une concentration de gaz sur base d'une phase et d'une amplitude de signaux de capteur qui sont obtenus à l'aide du capteur de gaz, et en fonction d'une information de pression et éventuellement d'une information de température fournies par le capteur de pression barométrique (10).

**12.** Aménagement de capteurs selon l'une des revendications 1 à 11, dans lequel l'aménagement de capteurs est entouré d'un boîtier qui présente en son intérieur un volume dans lequel se trouve l'aménagement de capteurs,

dans lequel le boîtier présente une ouverture de boîtier à travers laquelle un gaz à analyser peut arriver de l'extérieur du boîtier à l'aménagement de capteurs se trouvant dans le volume par un processus de diffusion,
dans lequel l'ouverture du boîtier présente une membrane qui est conçue pour protéger l'aménagement de capteurs contre une salissure et dans lequel la membrane est conçue pour permettre une diffusion d'un gaz à analyser.

**13.** Dispositif de capteurs, aux caractéristiques suivantes:

un canal de circulation (700),
dans lequel le canal de circulation (700) présente, dans une paroi, une ouverture; et
un aménagement de capteurs selon l'une des revendications 1 à 12, dans lequel l'aménagement de capteurs (100) est disposé de sorte que l'aménagement de capteurs soit connecté spatialement, à travers l'ouverture, à l'intérieur du canal de circulation (700) pour permettre un échange de gaz entre le l'intérieur du canal de circulation (700) et l'aménagement de capteurs.

**14.** Dispositif de capteurs selon la revendication 13, dans lequel le dispositif de capteurs est conçu de sorte que le cadre ferme hermétiquement vers l'extérieur un espace de mesure de gaz de l'ouverture de boîtier, dans lequel un laps de temps jusqu'à ce que l'équilibre de la concentration de gaz dans la zone du capteur de gaz diffère de tout au plus 0,5 % en volume de la concentration de gaz dans le canal de circulation (700) est inférieur à 10 ms.

**15.** Dispositif de capteurs selon l'une des revendications 13 ou 14, dans lequel le dispositif de capteurs est conçu de sorte qu'une chambre contenant l'aménagement de capteurs représente une zone de circulation calmée.

**16.** Dispositif de capteurs selon l'une des revendications 13 à 15, dans lequel le dispositif de capteurs présente un capteur de circulation (706), dans lequel le capteur de circulation (706) est disposé de manière à pouvoir déterminer une vitesse de circulation et/ou un débit de masse de gaz et/ou un débit volumique dans le canal de circulation (700).

**17.** Dispositif de capteurs selon l'une des revendications 13 à 16, dans lequel le dispositif de capteurs présente un deuxième capteur de pression barométrique qui est conçu pour mesurer une pression ambiante.

**18.** Dispositif de capteurs selon l'une des revendications 13 à 17, dans lequel le dispositif de capteurs est conçu pour reconnaître, sur base d'une information sur une vitesse de circulation d'air frais ou d'air frais enrichi par du gaz anesthésique dans le canal de circulation (700) et/ou sur base d'une information sur une direction de circulation d'air frais ou d'air frais enrichi par du gaz anesthésique dans le canal de circulation (700), un moment pour un étalonnage, et pour effectuer, en réaction à cela, un étalonnage du capteur de gaz thermique (20).

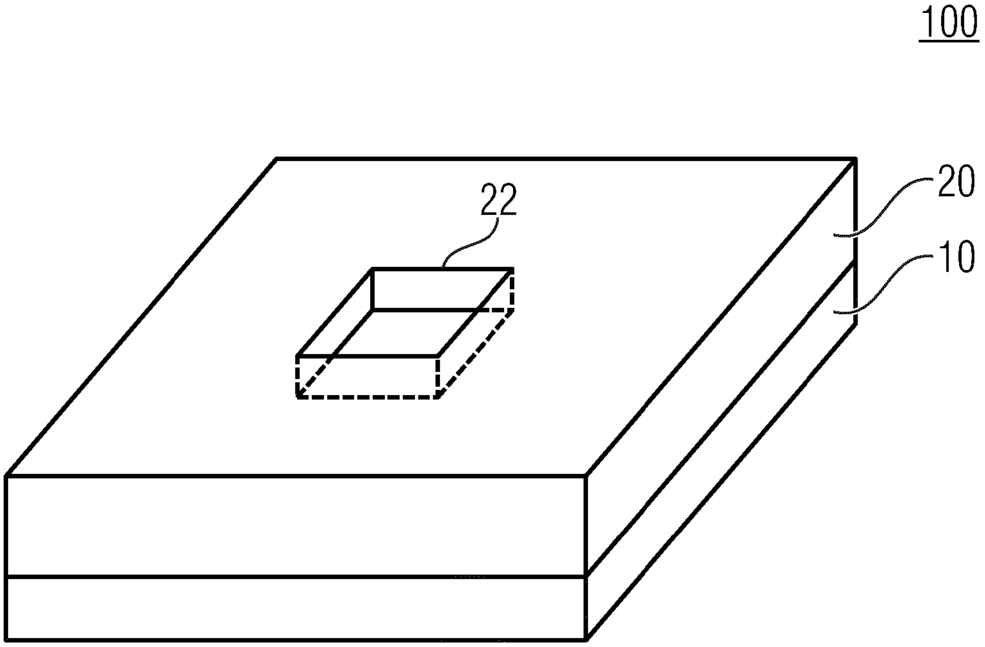


Fig. 1

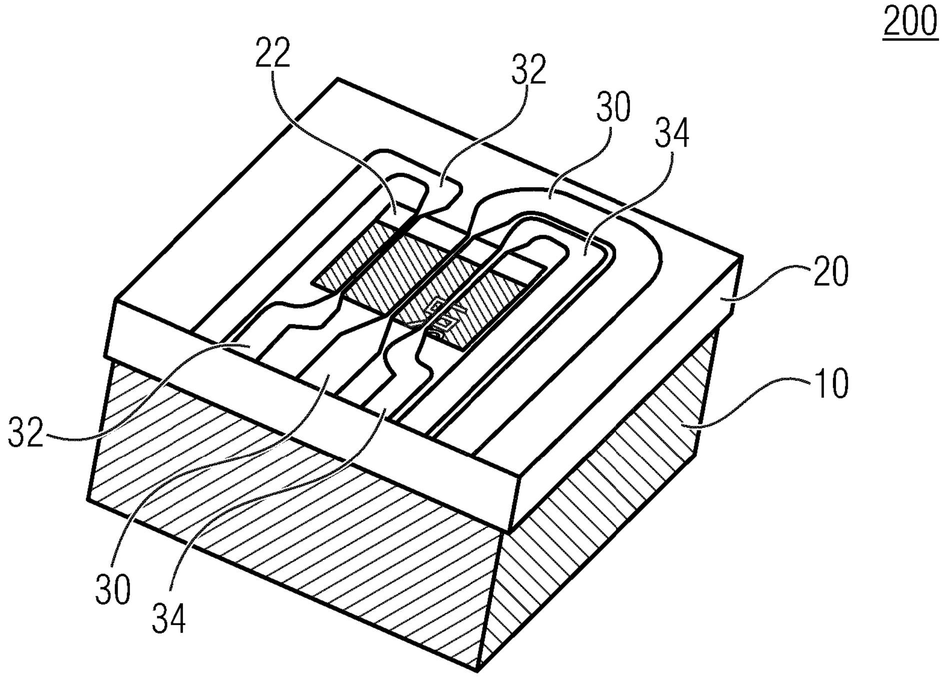


Fig. 2

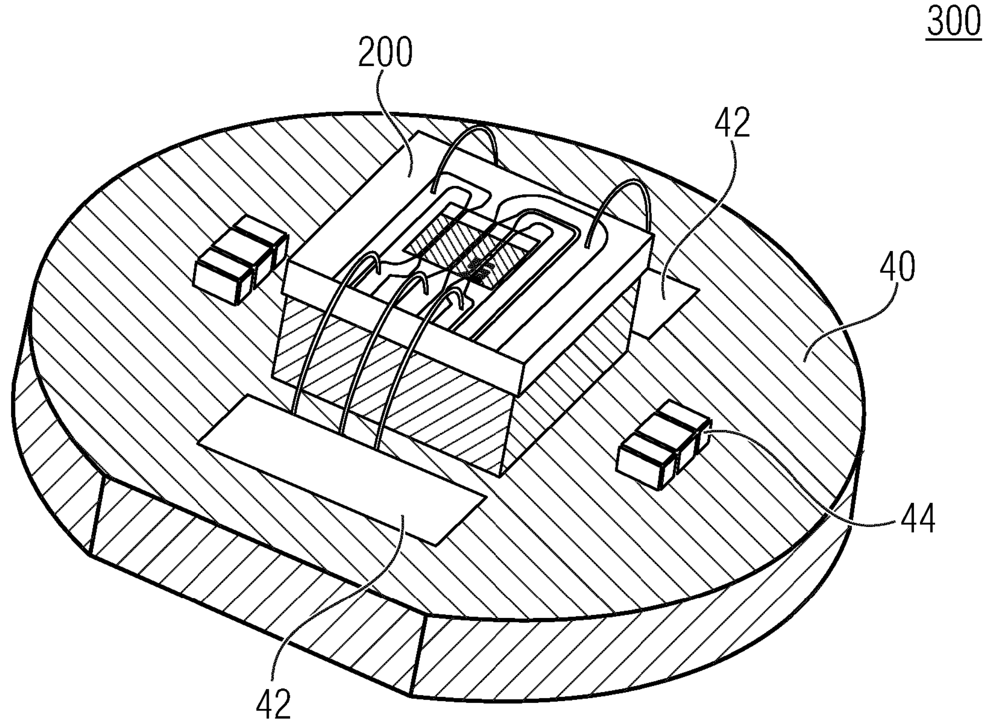


Fig. 3

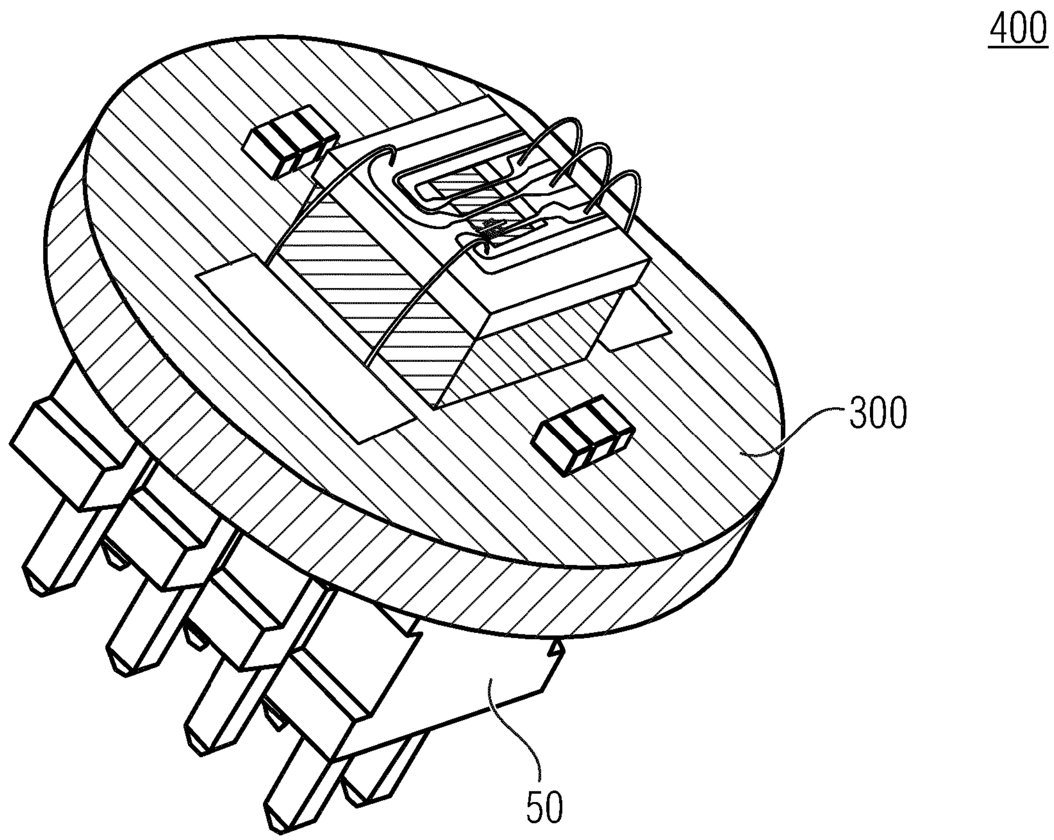


Fig. 4

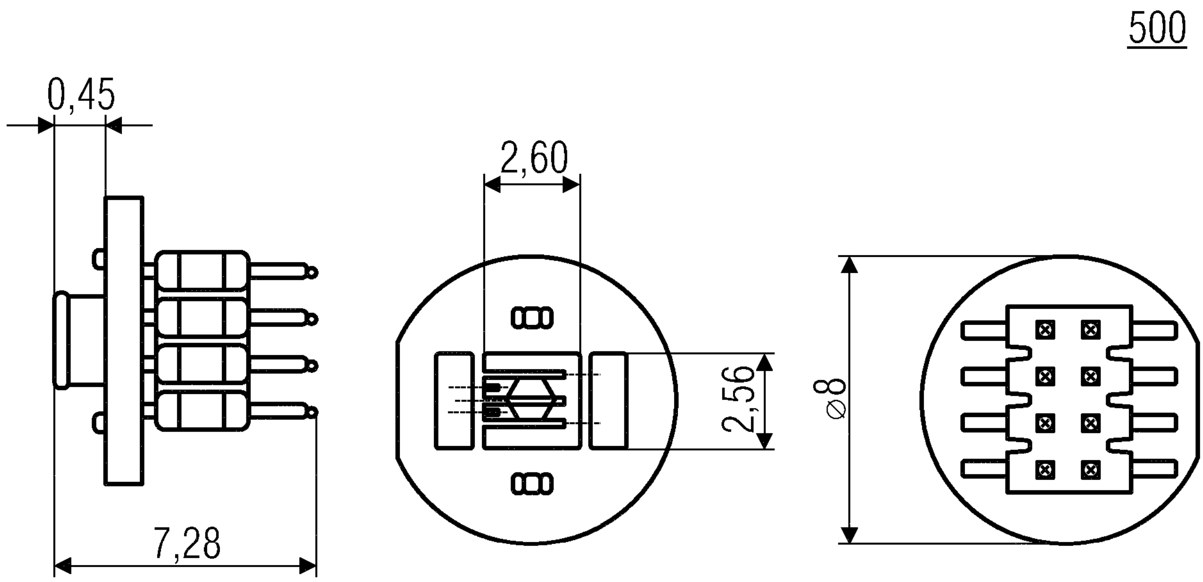


Fig. 5

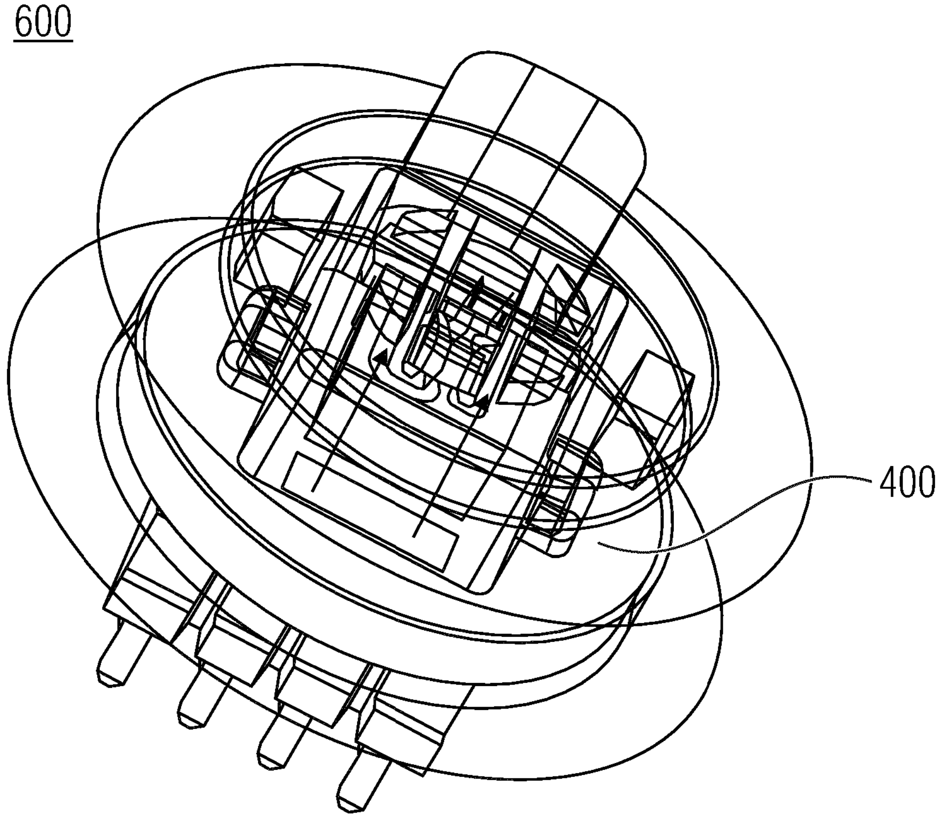


Fig. 6

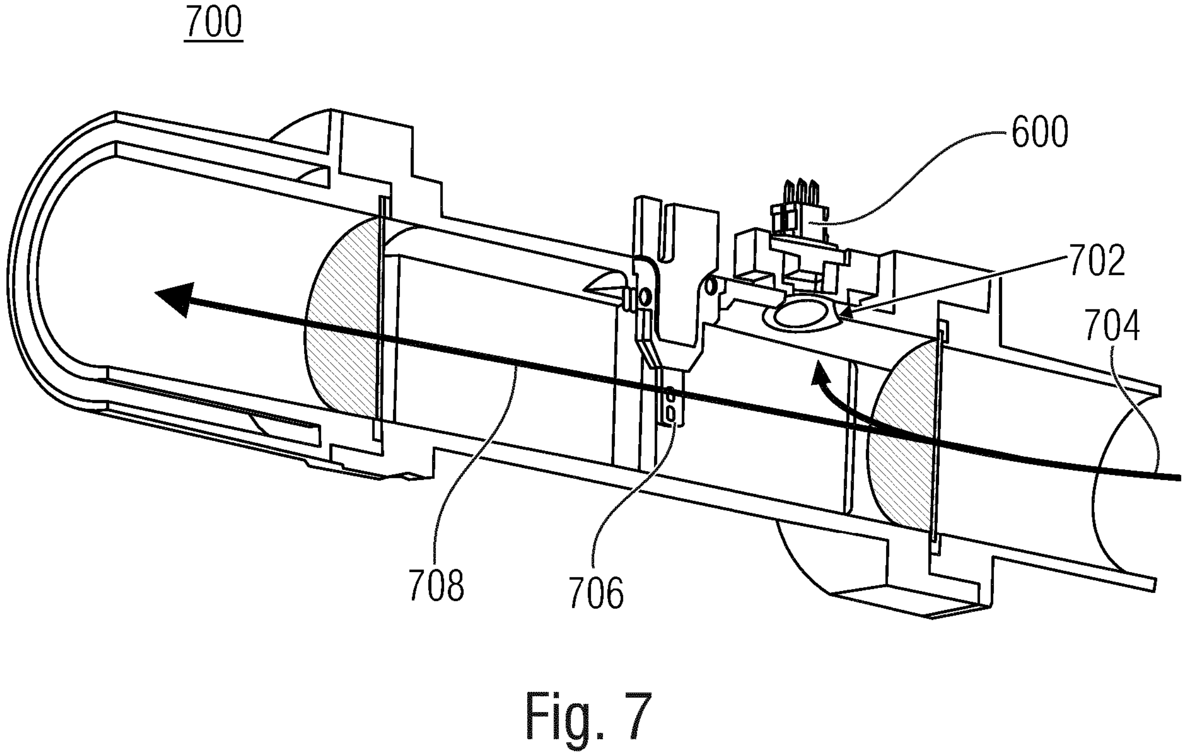


Fig. 7

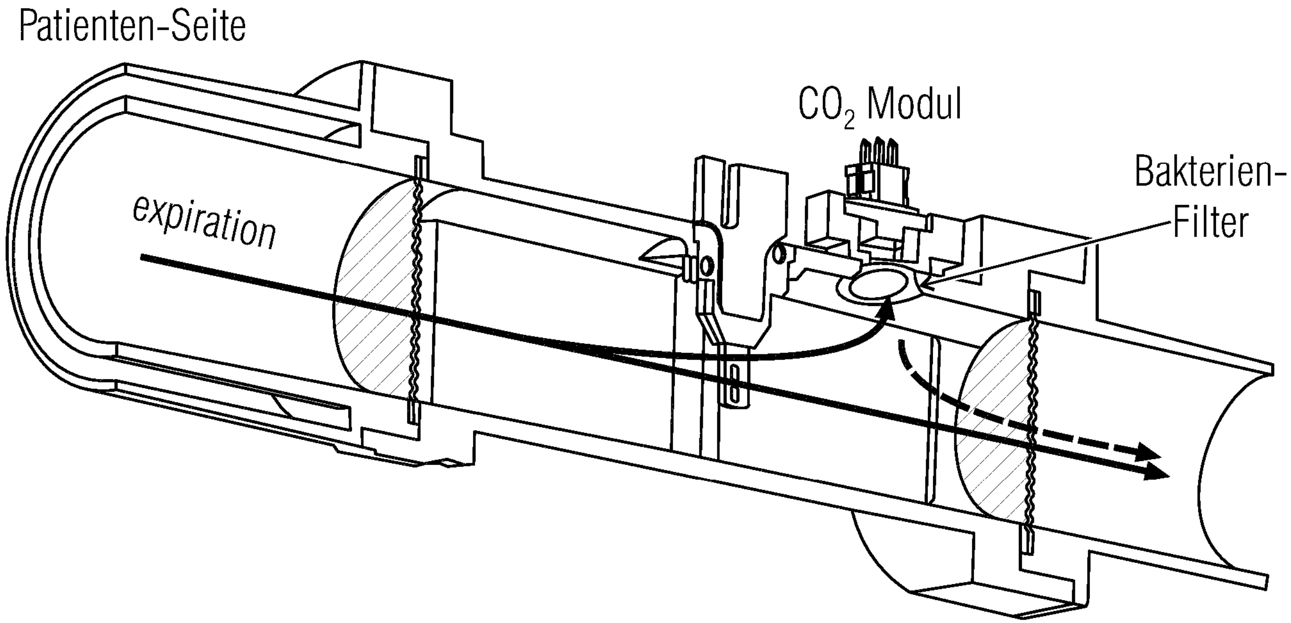


Während Exspiration: $Co_2$ Konzentration der Patienten in ausgeatmeter Luft
Diffusion der $CO_2$ Moleküle durch Bakterien Filter (als Beispiel: mit 1$\mu$m Maschenweite wird eine Diffusionszeit von 7,2ms für einen Konzentrationssprung auf 5vol% am Sensor benötigt).

Fig. 8

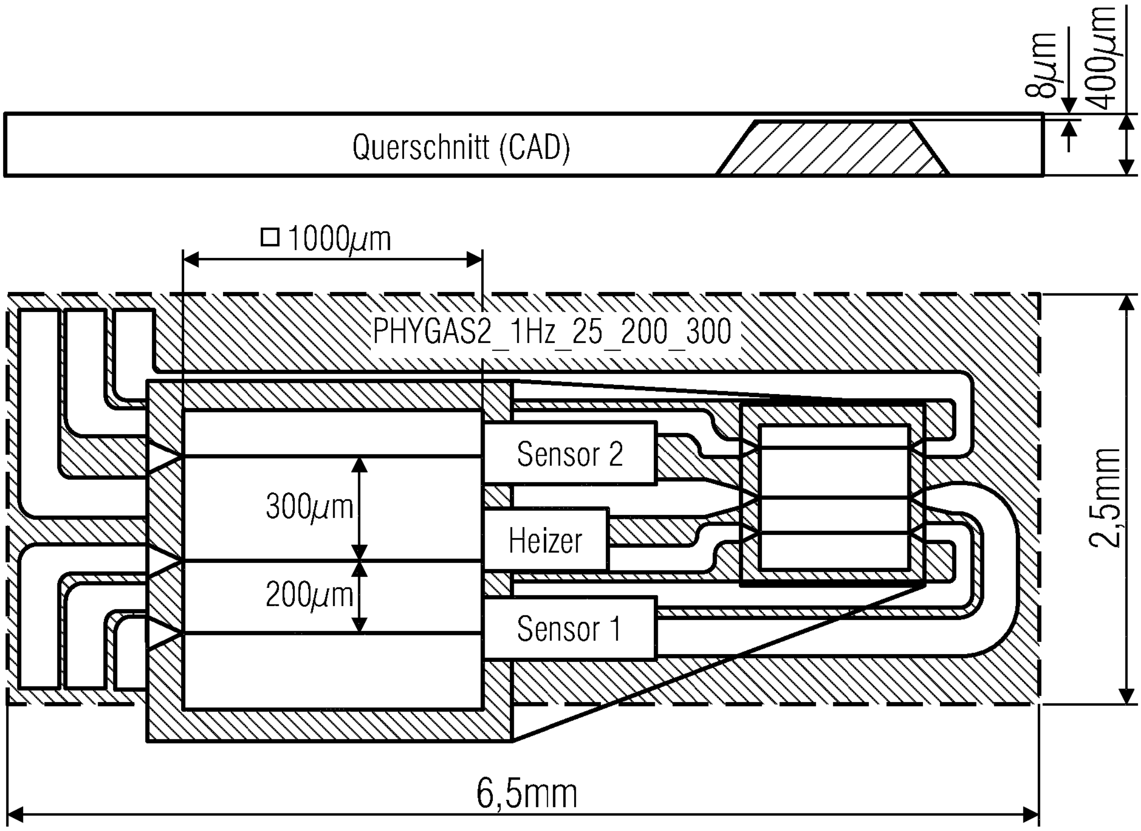

Querschnitt (CAD)
8µm
400µm
□1000µm
PHYGAS2_1Hz_25_200_300
Sensor 2
300µm
Heizer
200µm
Sensor 1
2,5mm
6,5mm


Fig. 9

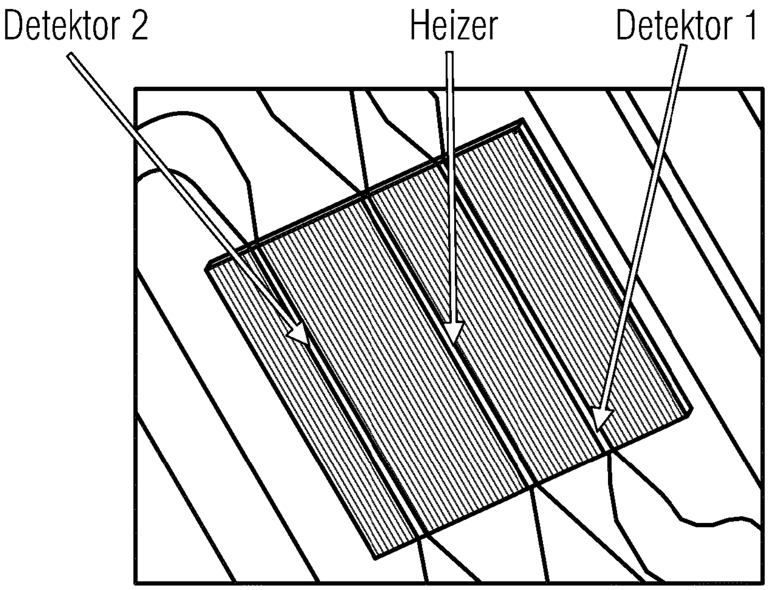

Detektor 2
Heizer
Detektor 1


Fig. 10

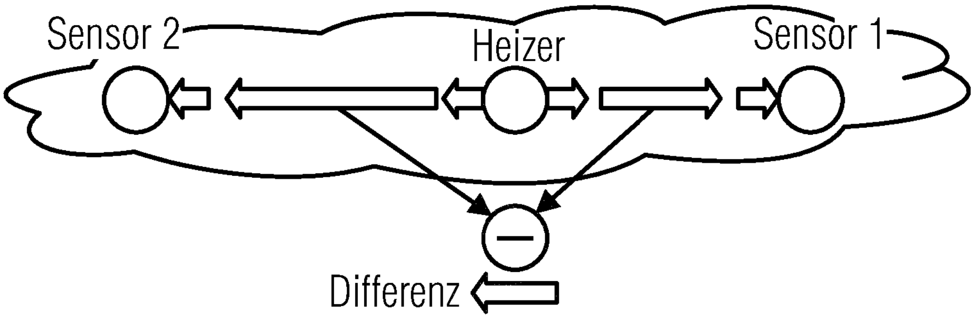
Sensor 2
Heizer
Sensor 1
Differenz

Fig. 11

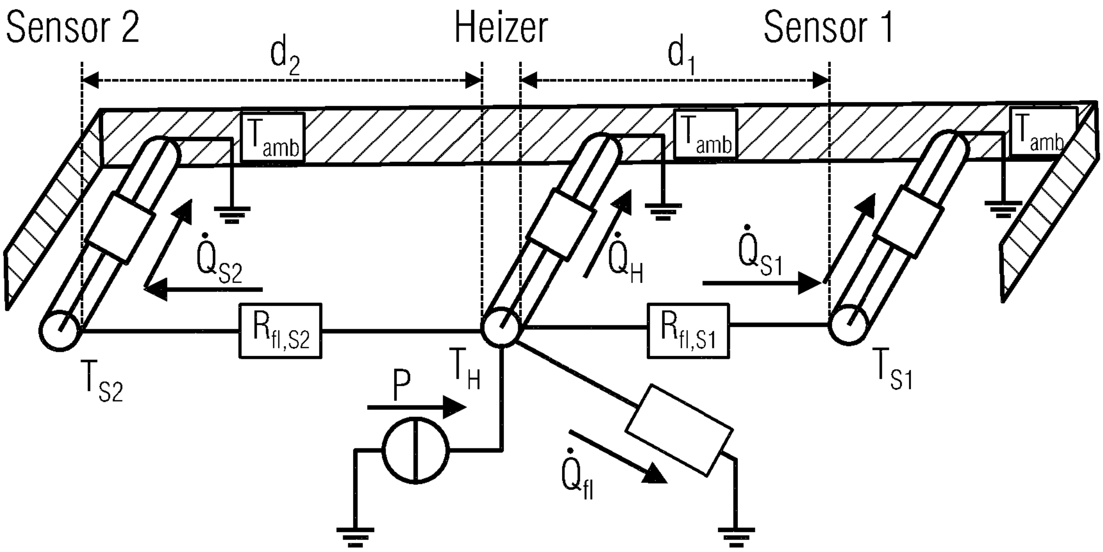
Sensor 2
Heizer
Sensor 1
$d_2$
$d_1$
$T_{amb}$
$T_{amb}$
$T_{amb}$
$\dot{Q}_{S2}$
$\dot{Q}_H$
$\dot{Q}_{S1}$
$R_{fl,S2}$
$R_{fl,S1}$
$T_{S2}$
$T_H$
$T_{S1}$
P
$\dot{Q}_{fl}$

Fig. 12

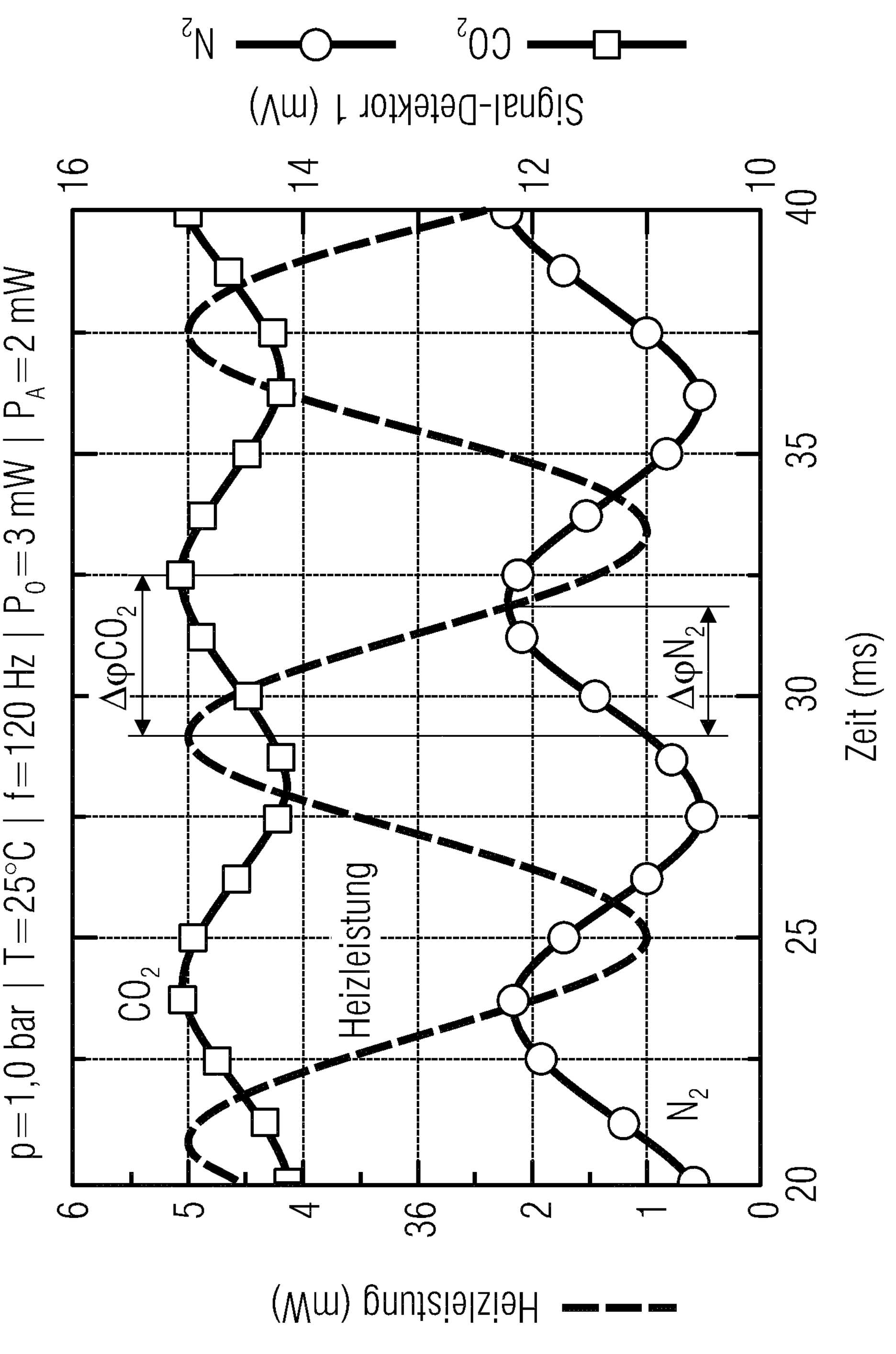
p = 1,0 bar | T = 25°C | f = 120 Hz | P0 = 3 mW | PA = 2 mW
Heizleistung (mW)
Signal-Detektor 1 (mV)
N2
CO2
Heizleistung
ΔφCO2
ΔφN2
Zeit (ms)
20
25
30
35
40
0
1
2
36
4
5
6
10
12
14
16

Fig. 13

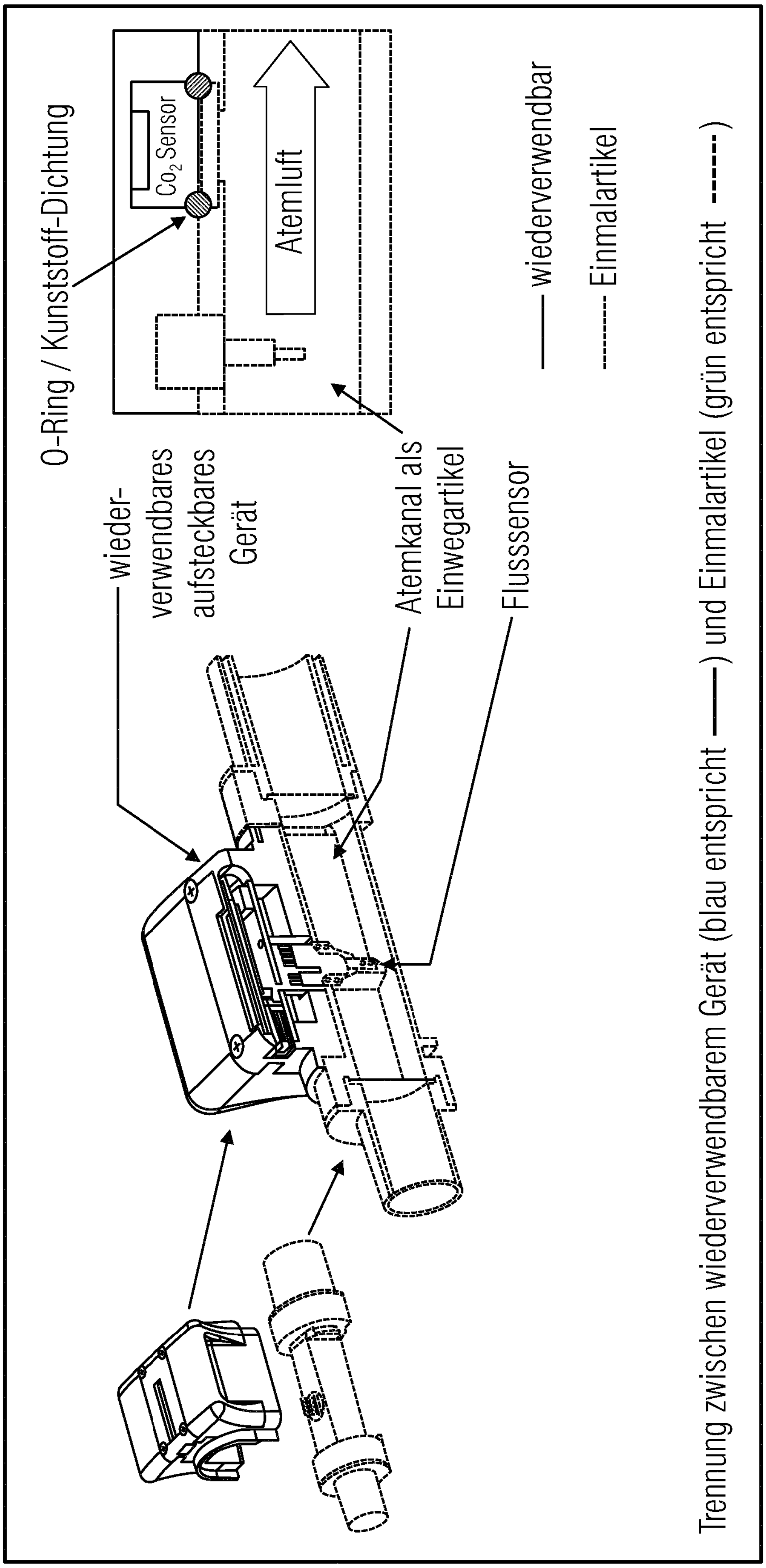
O-Ring / Kunststoff-Dichtung
$CO_2$ Sensor
Atemluft
wieder-
verwendbares
aufsteckbares
Gerät
Atemkanal als
Einwegartikel
Flusssensor
wiederverwendbar
Einmalartikel
Trennung zwischen wiederverwendbarem Gerät (blau entspricht ——) und Einmalartikel (grün entspricht ------)

Fig. 14

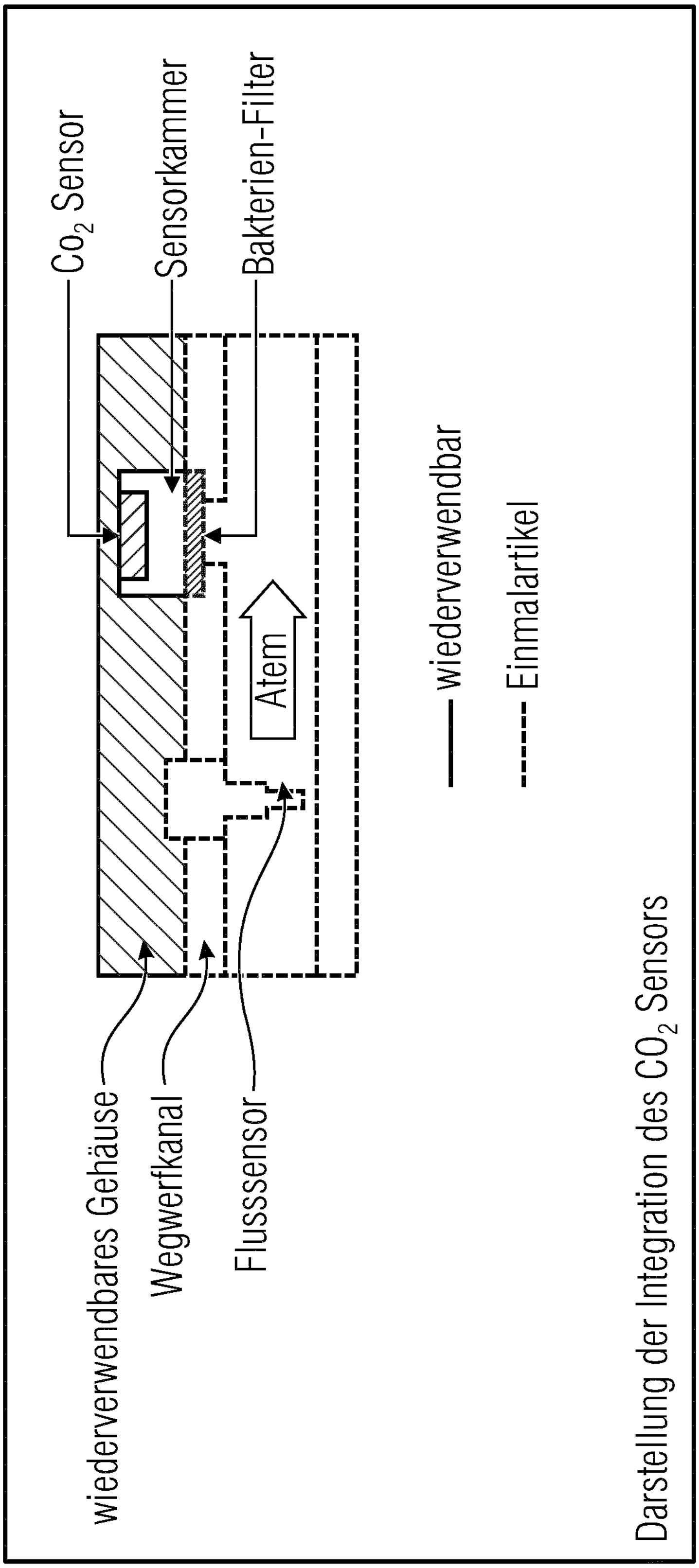
$Co_2$ Sensor
Sensorkammer
Bakterien-Filter
Atem
wiederverwendbar
Einmalartikel
wiederverwendbares Gehäuse
Wegwerfkanal
Flusssensor
Darstellung der Integration des $CO_2$ Sensors

Fig. 15

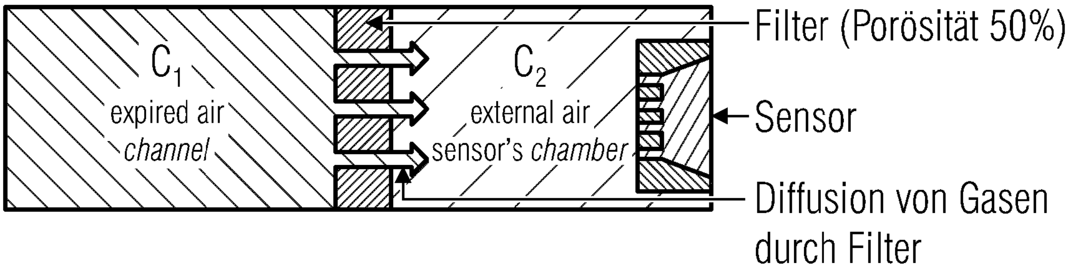


Einflussparameter auf die Diffusion nach dem Gesetz von Fick:

Expired air = ausgeatmete Luft
External Air = äußere Luft
Channel = Kanal
Sensor's chamber = Sensorkammer
Filter = Filter
Porosity = Porösität
Sensor = Sensor
Diffusion of gasses through the filter = Diffusion von Gasen durch den Filter

Fig. 16

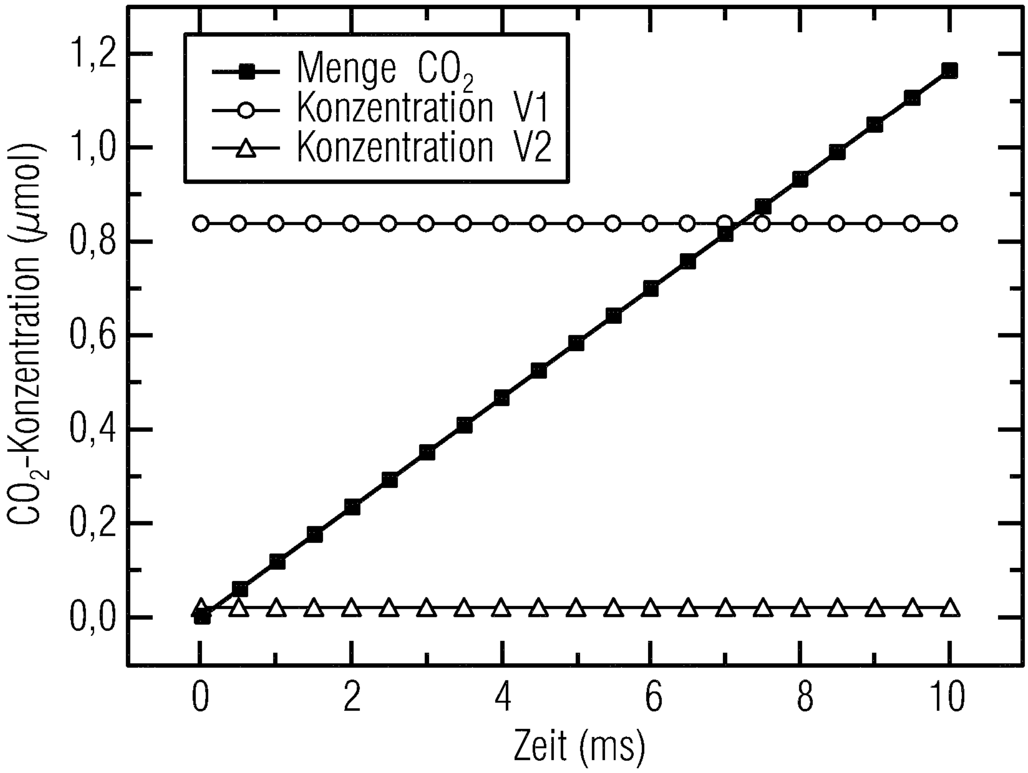


Fig. 17

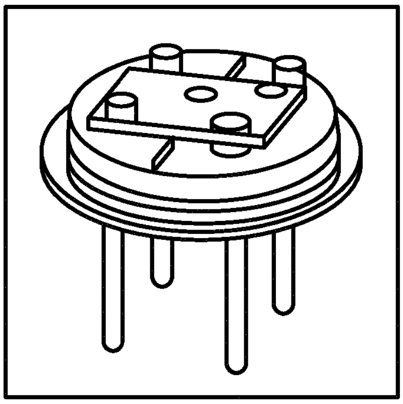

Metalloxid-Sensoren (MOX)

Fig. 18

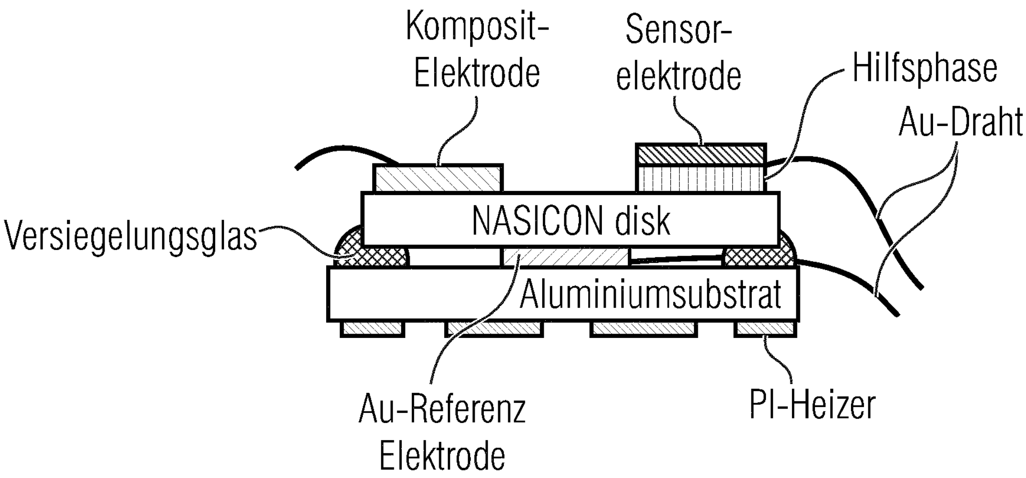


Elektrochemisch potenziometrische Sensoren (NASICON)

Fig. 19

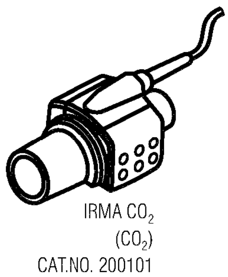


nichtdispersiver Infrarot-Sensor (NDIR)

Fig. 20

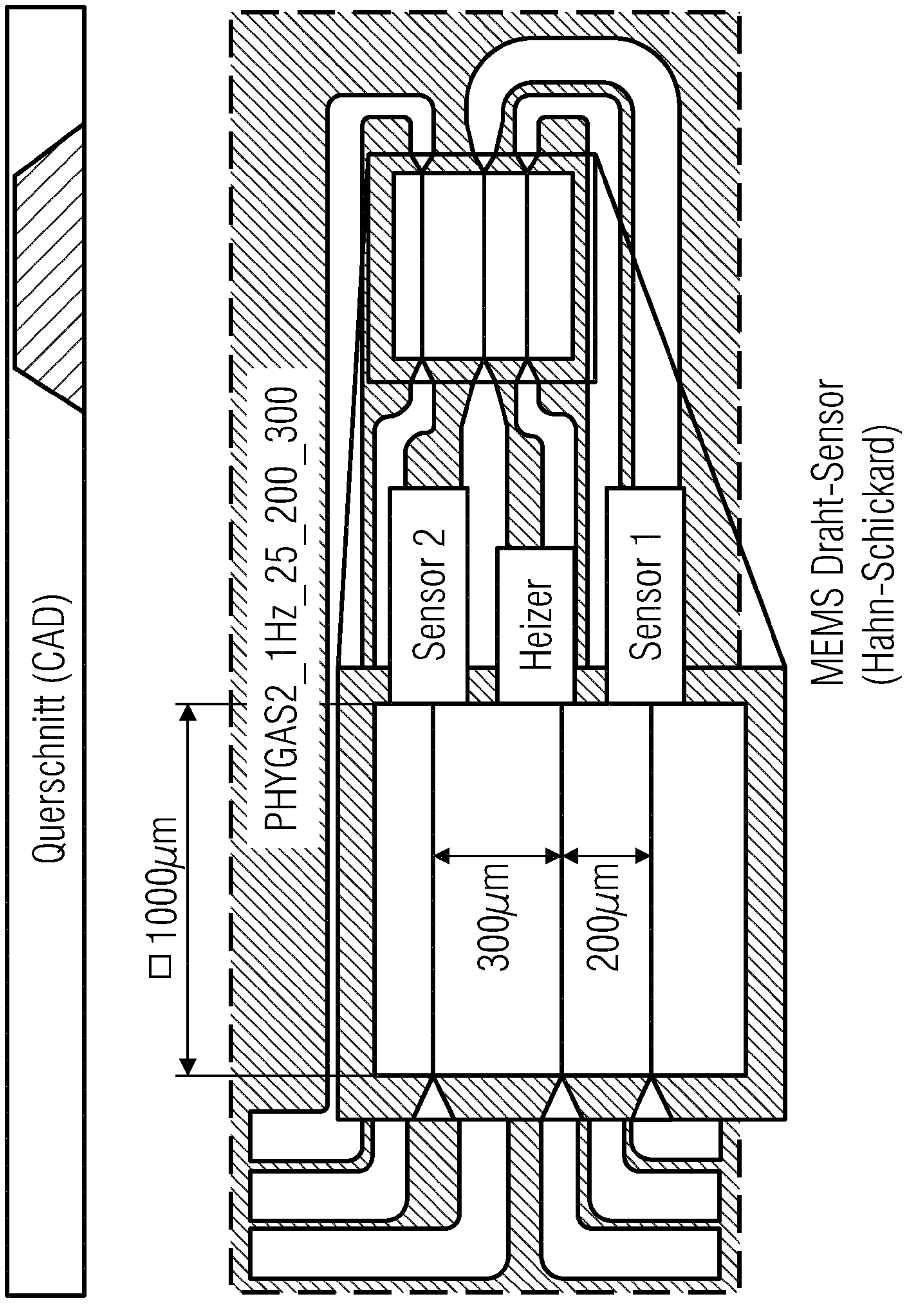
Querschnitt (CAD)
□1000μm
PHYGAS2_1Hz_25_200_300
Sensor 2
Heizer
Sensor 1
300μm
200μm
MEMS Draht-Sensor
(Hahn-Schickard)

Fig. 21

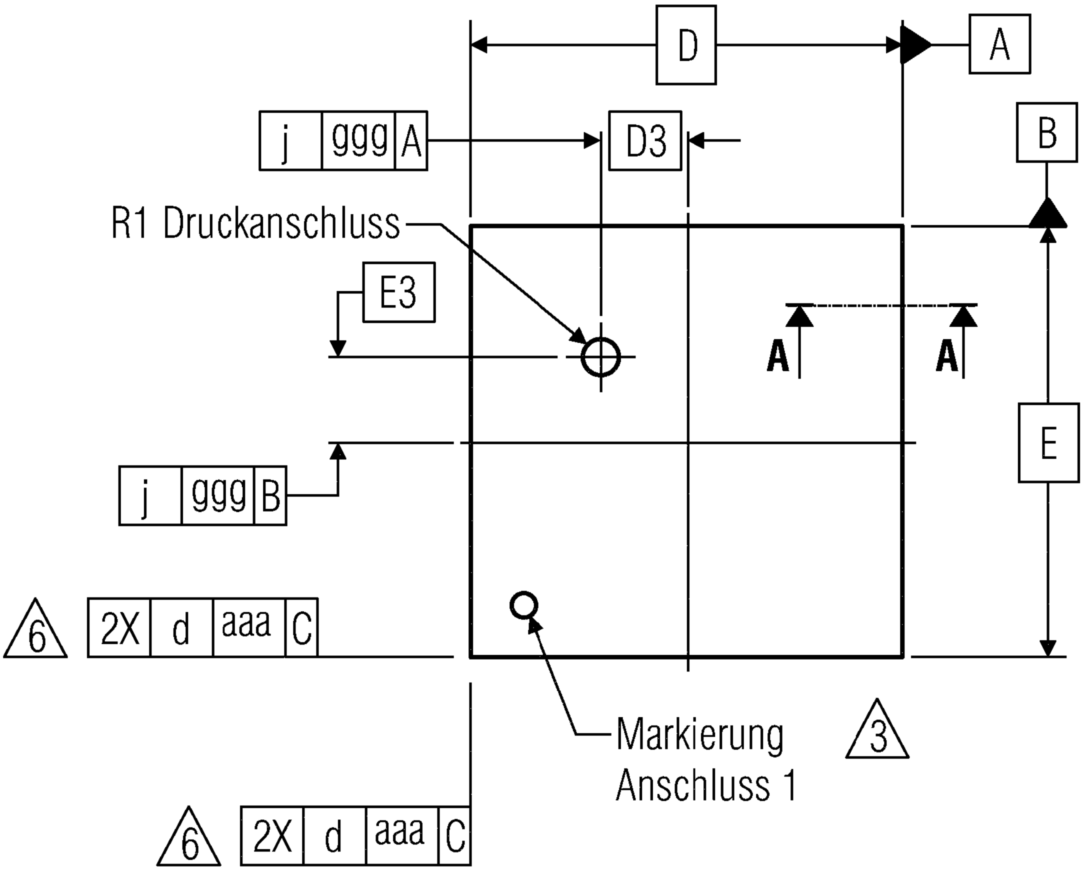


Draufsicht

Fig. 22

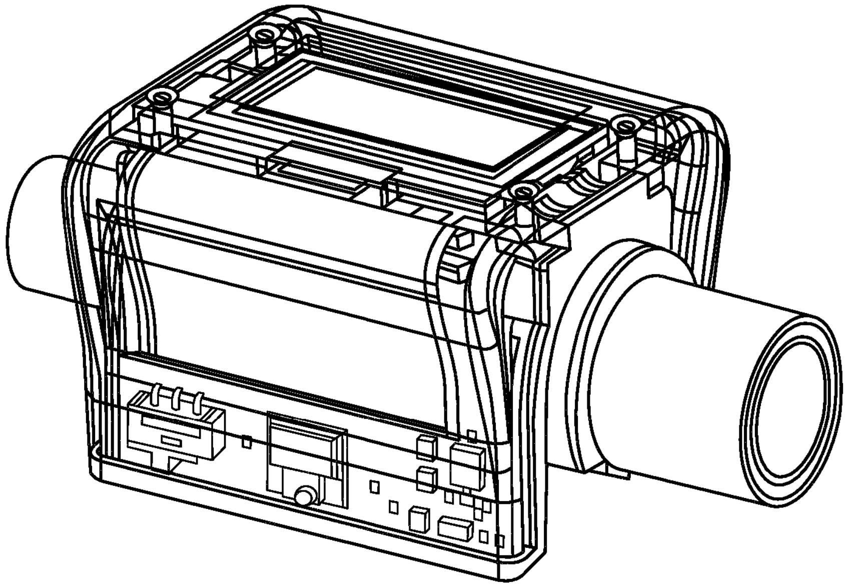

Fig. 23

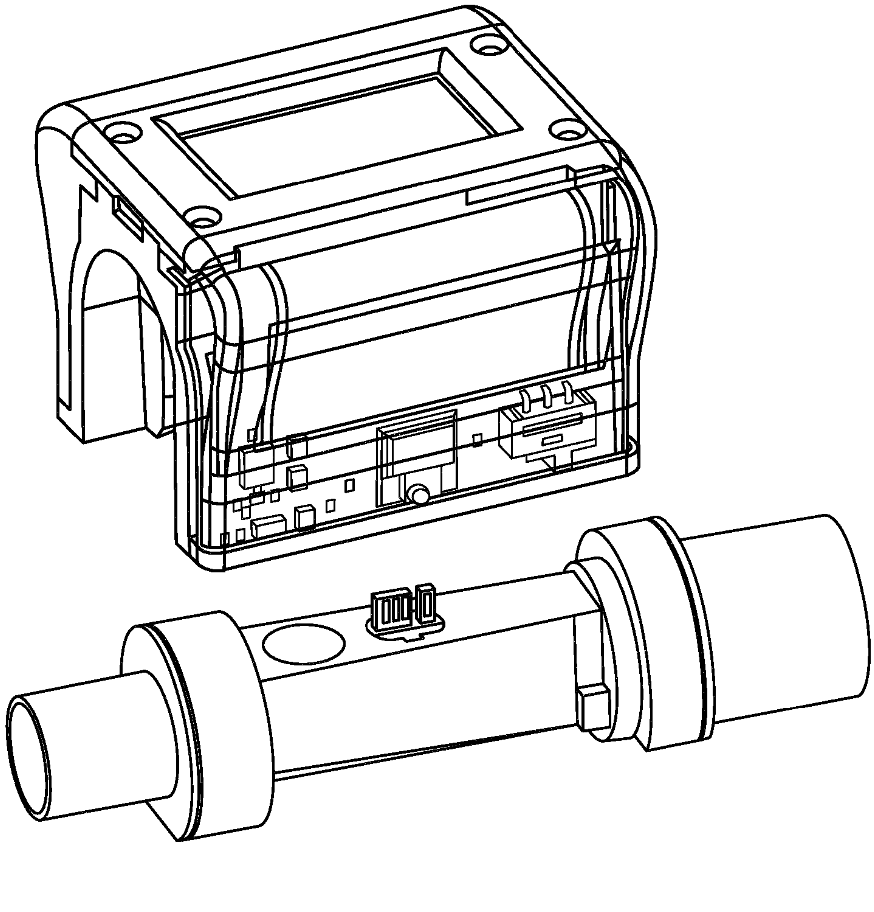

Fig. 24

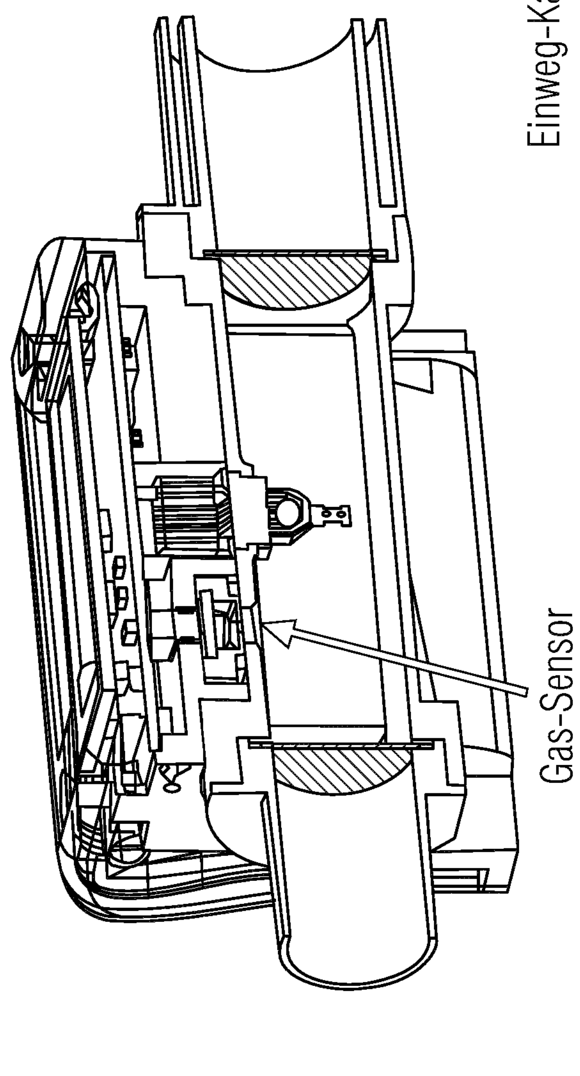


Fig. 25

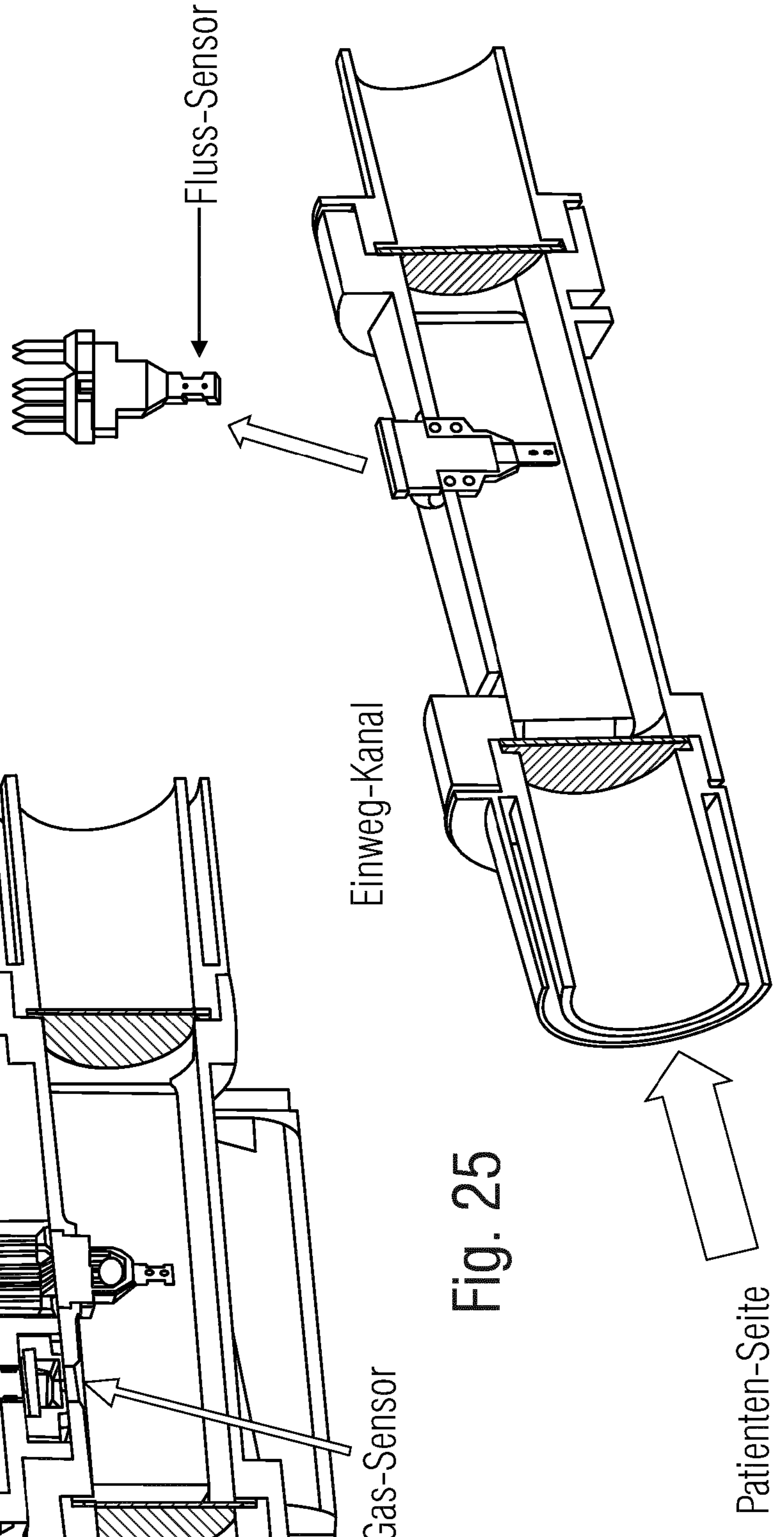


Fig. 26

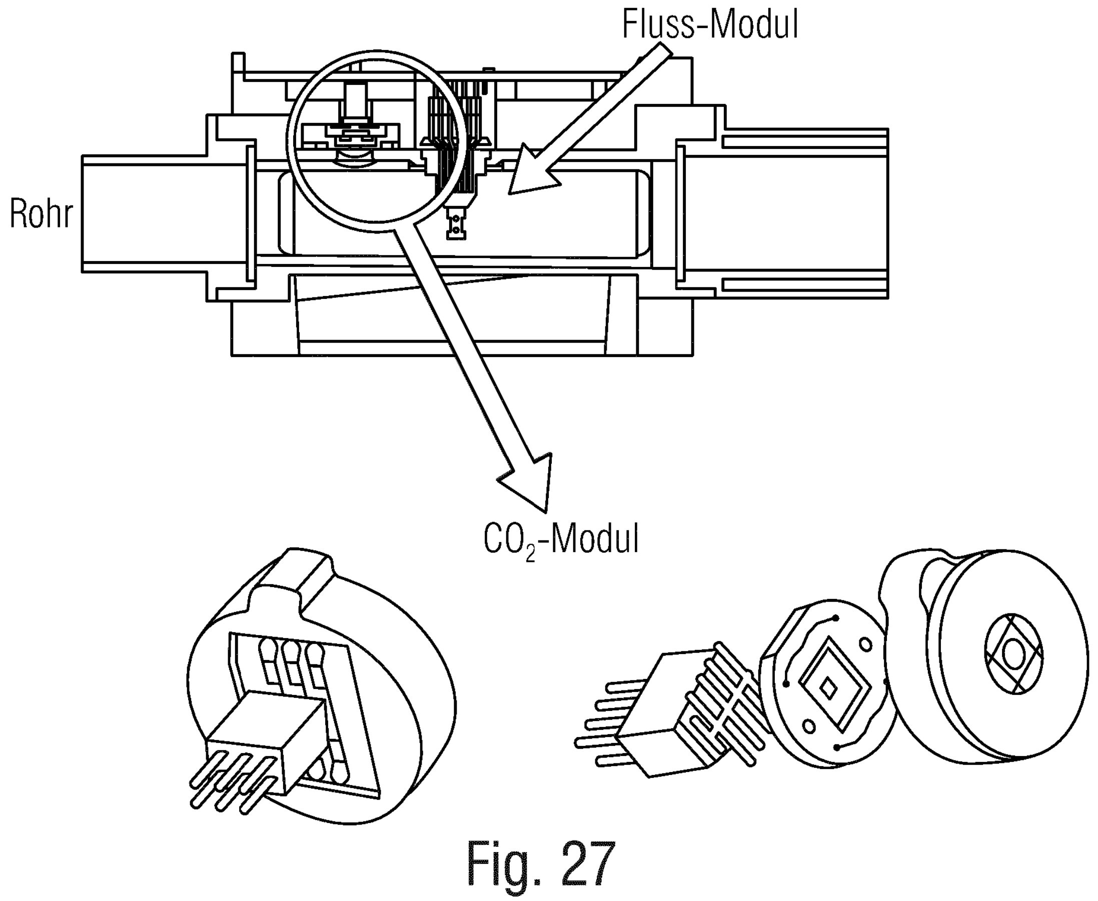
Fluss-Modul
Rohr
CO2-Modul

Fig. 27

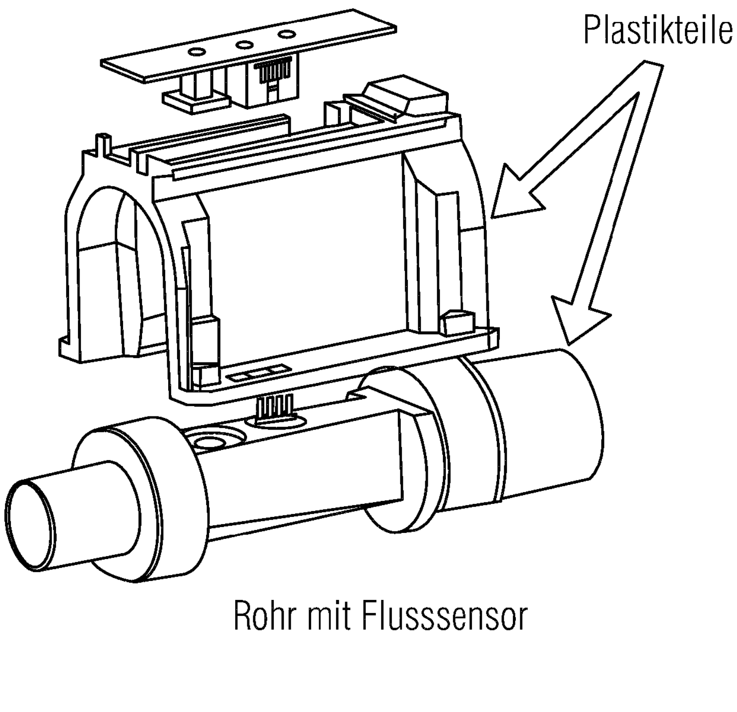
Plastikteile
Rohr mit Flusssensor

Fig. 28

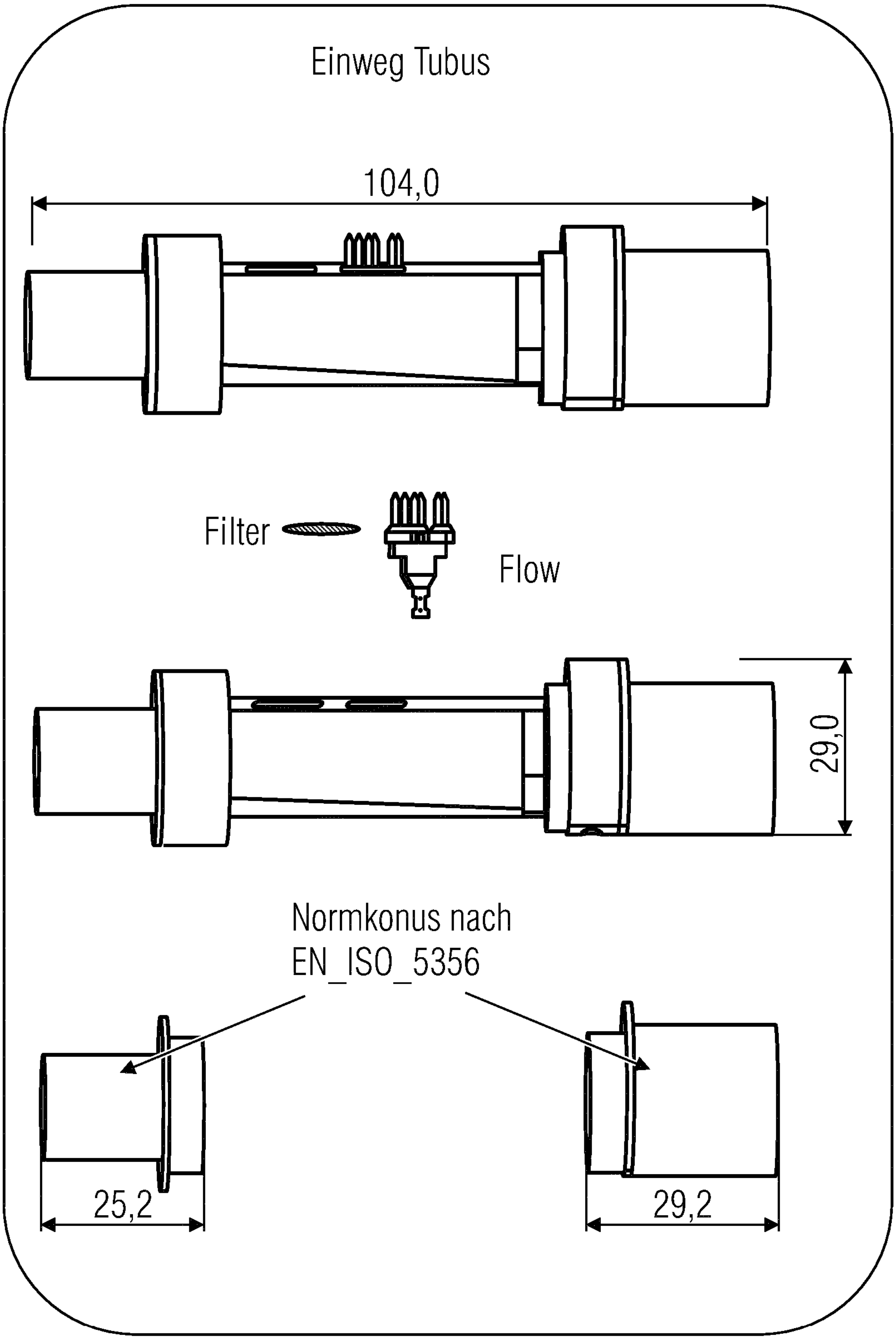
Einweg Tubus
104,0
Filter
Flow
29,0
Normkonus nach
EN_ISO_5356
25,2
29,2

Fig. 29

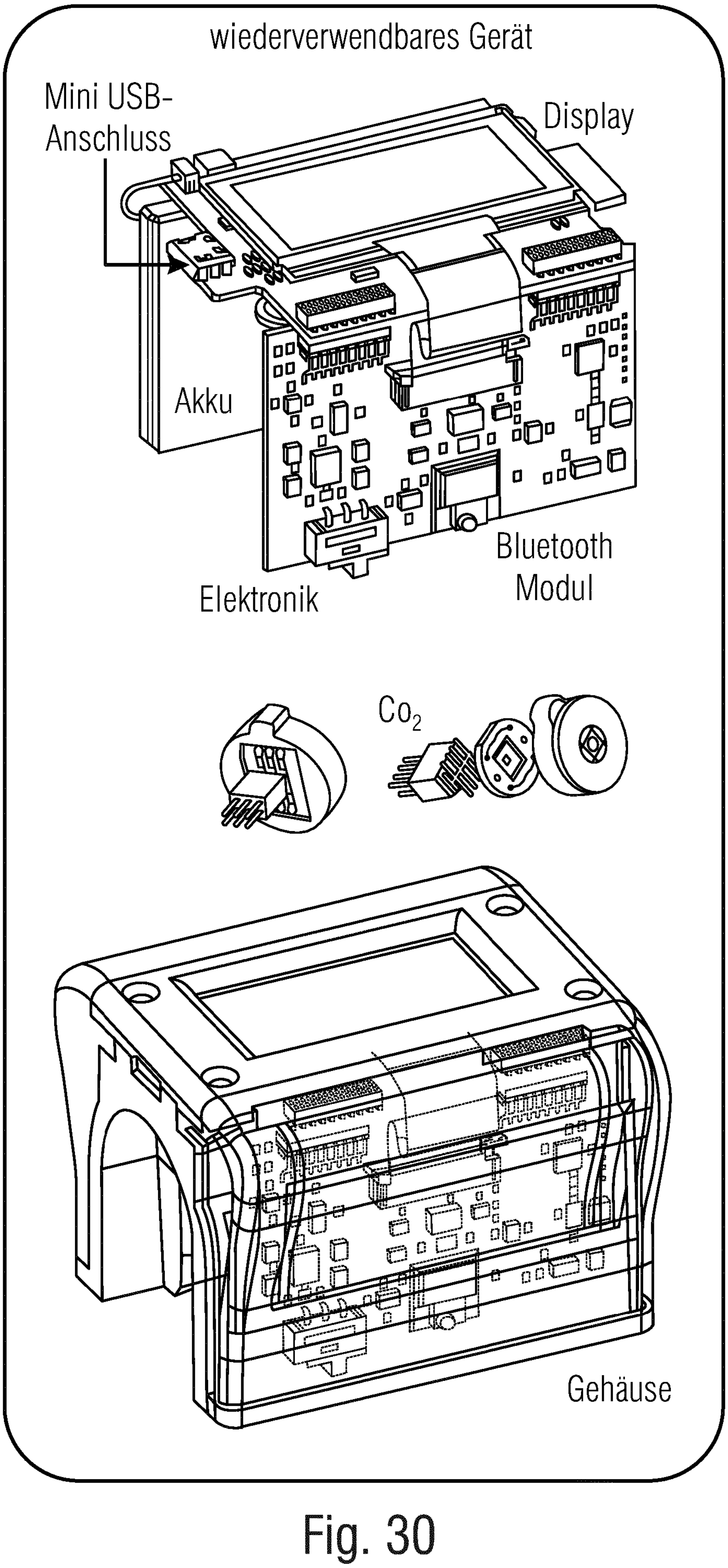
wiederverwendbares Gerät
Mini USB-
Anschluss
Display
Akku
Bluetooth
Modul
Elektronik
Co2
Gehäuse

Fig. 30

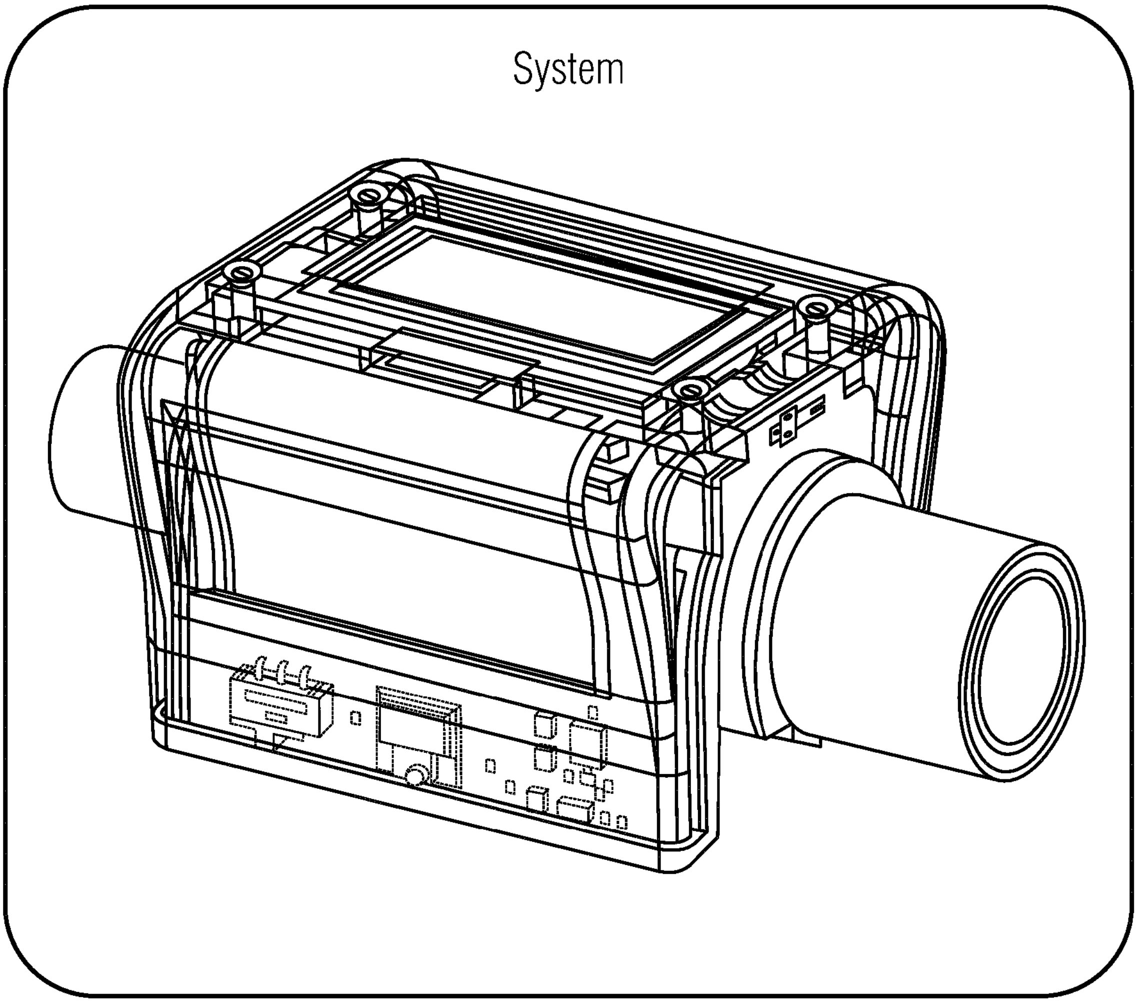


Fig. 31

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **VON JOHN VAN BAAR.** *Distributed thermal micro sensors for fluid flow,* ISBN 9-036-51828-8